# EUROPEAN PATENT SPECIFICATION

(11) **EP 2 544 693 B1**
(45) Date of publication and mention of the grant of the patent: **13.09.2017**
(21) Application number: 11754011.2
(22) Date of filing: 09.03.2011
(51) Int. Cl.: A61K 9/127, A61K 39/245, A61K 31/7088, A61K 39/00, A61K 39/12

(54) **A NOVEL MUCOSAL VACCINATION APPROACH FOR HERPES SIMPLEX VIRUS TYPE-2**
NEUER SCHLEIMHAUTIMPFUNGSANSATZ FÜR DEN HERPES-SIMPLEX-VIRUS VOM TYP 2
NOUVEAU PROCÉDÉ DE VACCINATION MUQUEUSE CONTRE LE VIRUS DE L'HERPÈS SIMPLEX DE TYPE 2

(30) Priority: 09.03.2010 US 312122 P
(43) Date of publication of application: 16.01.2013
(73) Proprietor: Biomedical Research Models, Inc., Worcester, MA 01606 (US)
(72) Inventor: YANG, Kejian, Worcester Massachusetts 01606 (US); GUBERSKI, Dennis L., Worcester Massachusetts 01606 (US)
(74) Representative: Walton, Seán Malcolm
(86) International application number: PCT/US2011/027751
(87) International publication number: WO 2011/112717

(56) References cited:
- US-A1- 2006 035 853
- US-A1- 2009 246 227
- US-B1- 6 451 320
- TIRABASSI REBECCA S ET AL: "A mucosal vaccination approach for herpes simplex virus type 2", VACCINE, vol. 29, no. 5, January 2011 (2011-01), pages 1090-1098, XP002699890, ISSN: 0264-410X
- DATABASE UNIPROT [Online] 03 March 2009 XP003028206 Database accession no. P03172
- CHU ET AL.: 'Antibody-mediated protection against genital herpes simplex virus type 2 disease in mice by Fc gamma receptor-dependent and -independent mechanisms' J REPROD IMMUNOL vol. 78, no. 1, June 2008, pages 58 - 67, XP022634643

## Description

### CROSS-REFERENCE TO RELATED APPLICATIONS

This application claims the benefit of U.S. Provisional Application No. 61/312,122, filed March 9, 2010, which is incorporated herein by reference in its entirety.

### STATEMENT OF GOVERNMENT SUPPORT

This invention was made with government support under Grant Numbers R43/AI063820-01, R43/AI063820-02, R44/AI063820-03, R44/AI063820-04 and R44/AI063820-05 awarded by NIAID, and Grant Numbers R31/CCR922413-01, R31/CCR924378-01, and R31/CCR924378-02 awarded by the Centers for Disease Control and Prevention. The government has certain rights in the invention.

### DESCRIPTION OF THE TEXT FILE SUBMITTED ELECTRONICALLY

The contents of the text file submitted electronically herewith are incorporated herein by reference in their entirety: A computer readable format copy of the Sequence Listing (filename: BMRI_005_01WO_SeqList_ST25.txt, date recorded: March 9, 2011, file size 22 kilobytes).

### FIELD OF THE INVENTION

The invention is related to the fields of immunology, virology, and vaccine development. In particular, the invention relates to a heterologous immunization protocol comprising an intramuscular priming dose comprised of a DNA vaccine encoding an antigen and an intranasal boosting dose comprised of the protein form of the antigen encapsulated in liposomes. This protocol is particularly effective in inducing both prophylactic and therapeutic immune responses to herpes-simplex virus.

### BACKGROUND OF THE INVENTION

Herpes simplex virus type 2 (HSV-2) is endemic in the human population and prevalent throughout the world. The World Health Organization estimated in 2003 that more than 300 million women and more than 200 million men were infected with HSV-2 (Cohen (2010) Science, Vol. 330: 304). According to the Centers for Disease Control and Prevention (CDC) approximately 20% of the US adult population is infected with HSV-2 (1), which can result in significant morbidity and psychological suffering. After initial replication in epithelial cells, virus enters neurons innervating the site of infection and enters latency. Periodically, HSV-2 will reactivate, replicate, form new viral particles and travel down the axon to the original infected site where it will undergo another round of lytic replication in the mucosal epithelium. Recurrences of genital ulcers typically occur 4 times per year (2). Asymptomatic shedding of virus in the absence of vesicle formation is also a common occurrence. As many as 70% of new cases of HSV-2 are reported to be acquired from partners with asymptomatic shedding (3) and it is estimated that HSV-2 infected women shed virus from the genital tract a total of 15-20% of days (4). Although HSV-2 generally results in mucosal lesions, HSV-2 infections involving other organs and surfaces are not uncommon (5). For example, HSV-2 infection can involve the central nervous
system where it induces the abrupt onset of fever and focal neurological symptoms. In addition, vertical transmission of virus from mother to infant and infections in immune compromised individuals can lead to viral encephalitis and/or dissemination of virus throughout the body (6). In the absence of treatment with nucleoside analogs, the mortality rate for these infants is 50% (6). In addition to causing primary disease on its own, HSV-2 is also a positive cofactor for HIV-1 transmission and has been associated with a 2-4 fold risk of acquiring HIV-1 (7).

While it should be feasible to develop protective immunity to HSV-2, a successful HSV-2 vaccine remains elusive. This is primarily due to the various ways in which HSV-2 interacts with the host immune system throughout its complicated replication cycle. Many different HSV-2 immunization strategies have been developed including the use of whole inactivated virus, live attenuated virus, live replication defective virus, subunit vaccines and DNA vaccines (Bernstein and Stanberry (1999) Vaccine, Vol. 17(13-14): 1681-1689; Krause and Straus (1999) Infect Dis Clin North Am., Vol. 13(1):61-81; McKenzie and Straus (1996) Rev Med Virol., Vol. 6:85-96). To date, the only vaccine candidate that demonstrated any efficacy in humans provided only limited protection from HSV-2, and solely in female patients that are seronegative for herpes simplex virus type 1 (HSV-1) (8). Recently published results from a follow-up trial reported that this subunit vaccine was largely ineffective, contradicting the results of the earlier trial (Cohen (2010) Science, Vol. 330: 304). Thus, a safe and effective vaccine for HSV-2 is still lacking.

Clinical trials and animal studies have indicated that any successful HSV-2 vaccine candidate must initiate protection in multiple forms. Humoral immunity is important for protection from extracellular virion particles during initial exposure, during vertical transmission of virus from mother to child and during reactivation of virus when extracellular particles are transmitted from neuron to epithelial cell (9, 10). Infections in B cell-deficient mice indicate that while HSV-specific antibody limits infection, other arms of the immune system are required to prevent infection (11). Cellular immunity is necessary for clearance of virus-infected epithelial cells during primary and recurrent infections, resolution of lytic infections in sensory ganglia and possibly in the prevention of reactivation (12-18). Depletion studies have demonstrated that protection against HSV-2 re-infection is primarily controlled by CD4⁺ T cells rather than CD8⁺ T cells or antibody (19-21). Further, long term immunity appears to be dependent upon mucosal rather than systemic immunization, highlighting the importance of local mucosal immune responses (22).

It is well known that a HSV-2 vaccine candidate capable of protecting against diseases may not completely contain virus infection and replication. Therefore, it has been a real challenge for a successful HSV-2 vaccine to provide protection against both primary HSV-2 infection-caused acute diseases and the subsequent development of latency and recurrence. In animal studies, some previous HSV-2 vaccine candidates have substantially reduced viral replication in the genital tract and significantly prevented the symptoms of disease resulting from primary infection. However, the immunity elicited by these vaccines can only partially protect against latent infection and recurrent disease (3-10). Vaccine induced host immune responses may act at one or more key steps to prevent or limit genital HSV infection. To prevent both acute disease and the establishment of latency, ideally immune responses elicited by a HSV-2 vaccine would be able to effectively contain the HSV-2 virus replication at the genital mucosae and successfully prevent virus transmission to sensory nerve endings. In order to obtain maximum protection against initial viral replication, it is most likely that a vaccine would need to induce broad and potent protective immunity, especially robust mucosal immune responses at genital sites. A therapeutic vaccine to treat those already infected with HSV-2 would ideally elicit immune responses capable of containing viral shedding and controlling clinical recurrences. At a minimum, a therapeutic vaccine should reduce the frequency, duration and severity of clinical recurrences and viral shedding.

Thus, there remains a clear need in the art for the development of a safe and effective therapeutic and prophylactic vaccine for HSV-2 due to the magnitude of the public health problem and the failure of antiviral drugs to prevent its spread.

### SUMMARY OF THE INVENTION

The present invention is based, in part, on the discovery of a heterologous immunization regimen that comprises a priming dose comprising a DNA vaccine encoding an HSV-2 antigen followed by a boosting dose comprising the protein form of the antigen encapsulated in liposomes. This immunization protocol induces high titers of serum antibodies, a Th1-biased immune response and potent mucosal immunity, which protects against initial infection by HSV-2 virus and prevents recurrence of the disease in infected subjects. Accordingly, the present invention provides a priming preparation and a boosting preparation for use in a method for eliciting a protective immune response against HSV-2 in a mammal the method comprising administering to the mammal a priming preparation comprises a vector encoding an HSV-2 antigen under the control of a promoter, wherein the antigen is a full-length HSV-2-gD glycoprotein, and wherein the sequence encoding the gD glycoprotein is codon-optimized for expression in mammalian cells; and a boosting preparation comprising the extracellular domain of the gD glycoprotein encapsulated in liposomes, thereby eliciting the protective immune response in the mammal wherein in the method the priming preparation is administered intramuscularly and the boosting preparation is administered mucosally. In one embodiment, the mammal is human.

In certain embodiments, the protective immune response against HSV-2 elicited by the methods of the invention is biased towards a Th1 type immune response. In another embodiment, the protective immune response comprises neutralizing antibodies in the serum and vaginal secretions. In some embodiments, the protective immune responses comprise a mucosal IgA response and/or a mucosal IgG response. In other embodiments, the protective immune responses comprise an increase in the serum level of antigen-specific IgG and IgA compared to the serum level of antigen-specific IgG and IgA prior to the administration of the boosting preparation. In still other embodiments, the protective immune response comprises an increase in viral clearance (*e.g.* greater than 50%, greater than 75%, greater than 85%, greater than 90%, greater than 95% viral clearance) and /or leads to a complete (*e.g*. 100%) viral clearance.

Also described is a method for treating an HSV-2 infection in an animal. In the invention, the priming preparation is administered intramuscularly and the boosting preparation is administered mucosally (*e.g*., intranasally, orally, or intravaginally). In one particular embodiment, the boosting preparation is administered intranasally. One or more symptoms of HSV-2 infection can be ameliorated in the mammal following administration of the boosting preparation. For instance, in one embodiment, the recurrence of herpatic lesions is reduced and/or prevented in the mammal as compared to an untreated animal. In one particular embodiment, the mammal is human.

In the invention, the vector in the priming preparation is a vector encoding the HSV-2 antigen under the control of a promoter, such as a cytomegalovirus promoter. In the invention, sequence encoding the HSV-2 antigen (*e.g.* gD glycoprotein) is codon-optimized for expression in mammalian cells, particularly human cells.

In another aspect of the invention, the HSV-2 antigen in the boosting preparation is encapsulated in anionic liposomes. The liposomes may have an average diameter of about 0.5-5 µm. In certain embodiments, the HSV-2 antigen encapsulated in liposomes in the boosting preparation is a gD glycoprotein, which may be the full-length protein or an immunogenic fragment thereof. In one particular embodiment, the HSV-2 antigen is an extracellular domain of a gD glycoprotein (*e.g.* amino acids 1-314 of the gD glycoprotein). In one embodiment, the liposomes used in the boost preparations consist of lipids, *i.e.* the liposomes do not contain additional proteins, ligands, or adjuvants. In another embodiment, the liposomes are non-fusogenic liposomes *(i.e.* do not contain any viral proteins incorporated into the liposomal membrane).

Also described are kits for eliciting a protective or therapeutic immune response against HSV-2 in an animal (*e.g*. human). In one case, the kit comprises a first immunizing component comprising a nucleic acid encoding an HSV-2 antigen (e.g. gD glycoprotein) and a second immunizing component comprising the antigen encapsulated in liposomes. In some cases, the first immunizing component is formulated for intramuscular administration, and the second immunizing component is formulated for intranasal administration. In certain cases, the kit further comprises an instruction to administer to the animal the first immunizing component followed by administering the second immunizing component to elicit the protective or therapeutic immune response in the animal. In some cases, the animal is human. In one particular case, the human is infected with HSV-2. In another case, the human is at risk of infection with HSV-2.

Also described is a method for providing passive, protective immunity against HSV-2 in a mammal in need thereof. In one case, the method comprises isolating serum from a donor mammal immunized by the heterologous immunization methods described herein and administering the isolated, immune serum to a recipient mammal, wherein the recipient mammal is thereby protected from HSV-2 infection. In some cases, the isolated immune serum comprises HSV-2 antigen-specific IgG and IgA antibodies and/or HSV-2 neutralizing antibodies. In one case, the method further comprises isolating CD4⁺ T lymphocytes from the donor mammal and administering the isolated CD4⁺ T lymphocytes to the recipient mammal. The donor and recipient mammals may both be human. The recipient mammal, in some cases, is at risk of HSV-2 infection.

### BRIEF DESCRIPTION OF THE DRAWINGS

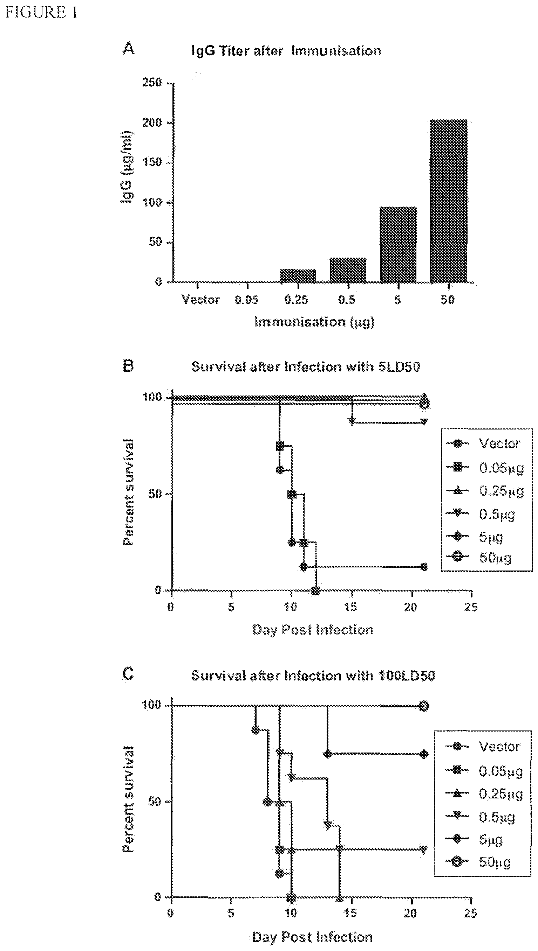

**Figure 1****.** Antibody responses and survival curves after DNA immunization. Adult female Balb/c mice were immunized i.m. with either vector control or with 0.05, 0.25, 0.5, 5 or 50 µg gD DNA vaccine. Three weeks after immunization, serum IgG levels were measured from pooled samples (A). 6 weeks after immunization mice were injected with medroxyprogesterone and infected with either 5 LD50 **(B)** or 100 LD50 of HSV-2 (C). Mice were observed daily for clinical scores and mortality (n = 8 for naïve and 0.5 µg DNA; n = 4 for 0.05, 0.25, 5 or 50 µg DNA).

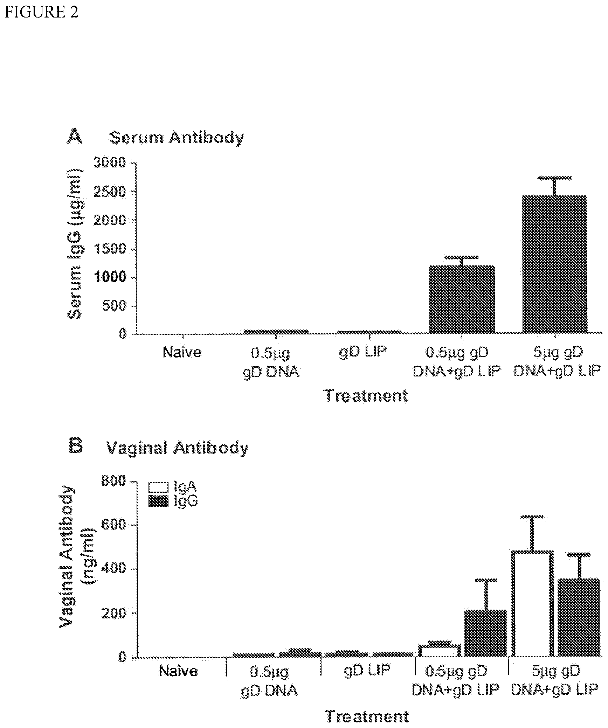

**Figure 2****.** Serum and vaginal antibody responses to heterologous immunization. Female Balb/c mice were either left untreated (naive), immunized with 0.5 µg of gD DNA vaccine i.m. on days 0 and 2 (gD DNA), 15 µg of gD protein encapsulated in liposomes (gD LIP) or 0.5 µg or 5 µg of gD DNA i.m. on days 0 and 2 followed by gD LIP in. 3 weeks after the last DNA immunization (gD DNA+ gD LIP). Serum and vaginal antibody responses were measured 2 weeks after the liposome immunization. Average gD-specific serum IgG (A) and vaginal antibody **(B)** responses ±STEM are shown. n = 2, 5, 5 and 8 for naïve, gD DNA, gD LIP and gD DNA+ gD LIP groups, respectively.

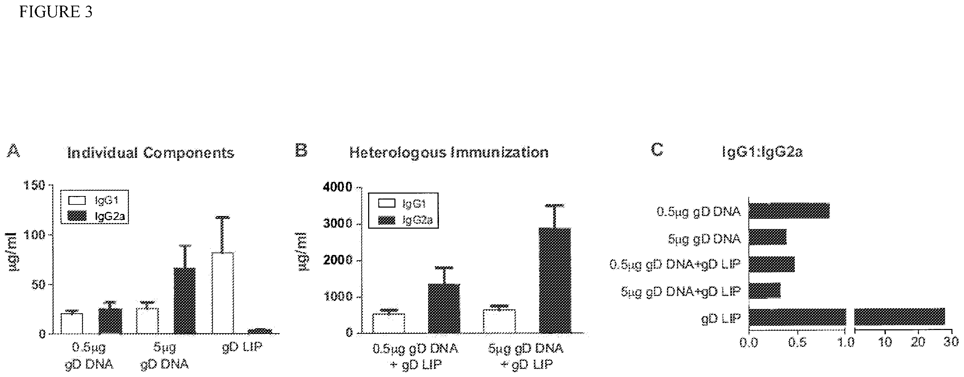

**Figure 3****.** IgG1:IgG2a ratios in immunized mice. Female Balb/c mice were immunized with either 0.5 µg or 5 µg of gD DNA vaccine alone i.m. on days 0 and 2 (gD DNA), 15 µg of gD protein encapsulated in liposomes alone (gD LIP) or 0.5 µg or 5 µg of gD DNA i.m. on days 0 and 2 followed by gD LIP i.n. 3 weeks after the last DNA immunization (gD DNA+ gD LIP). Serum gD-specific IgG1 and IgG2a responses were measured 2 weeks after the liposome immunization. Average gD-specific IgG responses ±SEM are shown. **A.** Average responses after vaccination with individual components. **B.** Average responses after heterologous immunization. n = 8 for 0.5 µg gD DNA alone, 0.5 µg gD DNA+ gD LIP and 5 µg gD DNA+ gD LIP; n = 7 for gD LIP alone; n = 6 for 5 µg gD DNA alone. C. IgG1:IgG2a ratio.

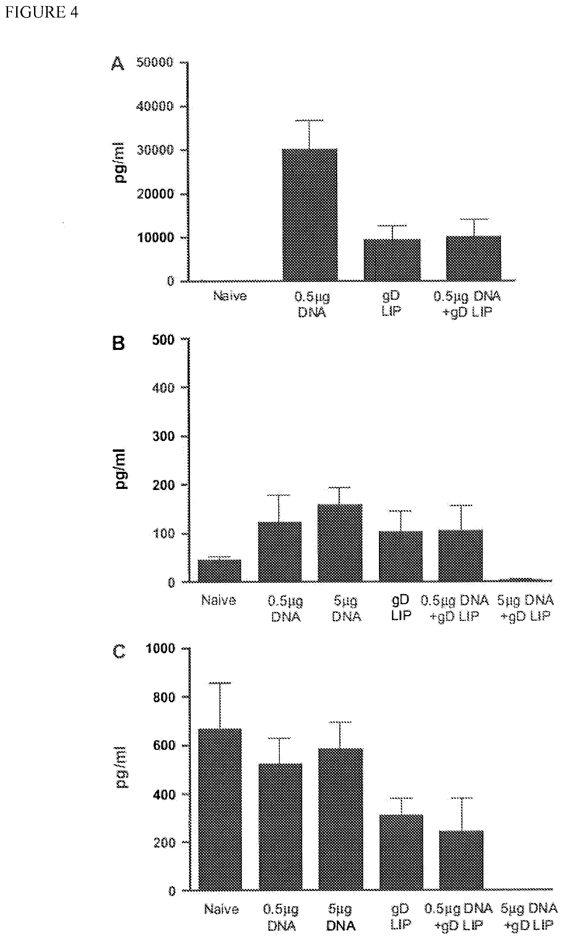

**Figure 4****.** IFN-γ production in vaccinated and infected mice. Female Balb/c mice were either left untreated, immunized with 0.5 µg or 5 µg of gD DNA vaccine i.m., 15 µg of gD protein encapsulated in liposomes or 0.5 µg or 5 µg of gD DNA i.m. followed by gD LIP i.n. 3 weeks after the last DNA immunization. Two weeks after the last immunization, animals were injected with progesterone and inoculated with 100 LD50 of HSV-2. **A.** On day 10 post infection, spleens were isolated from one naïve animal, 3 animals immunized with 0.5 µg of DNA, 5 animals immunized with gD LIP or 4 animals immunized with 0.5 µg DNA+ gD LIP and used to make whole cell suspensions. Cells were incubated with 10 µg/ml gD protein for 48 h prior to collecting cell supernatants. IFN-γ levels in the supernatants were measured using the "Ready-Set-Go" cytokine ELISA kit from eBioscience. **B and C.** Vaginal samples were collected on days 3 **(B)** and 5 **(C)** post infection from naive mice (n = 6) and mice immunized with 0.5 µg or 5 µg gD DNA (n = 6), gD LIP (n=6) or 0.5 µg or 5 µg gD DNA+ gD LIP (n = 5). IFN-γ levels in the samples were measured using the "Ready-Set-Go" cytokine ELISA kit from eBioscience.

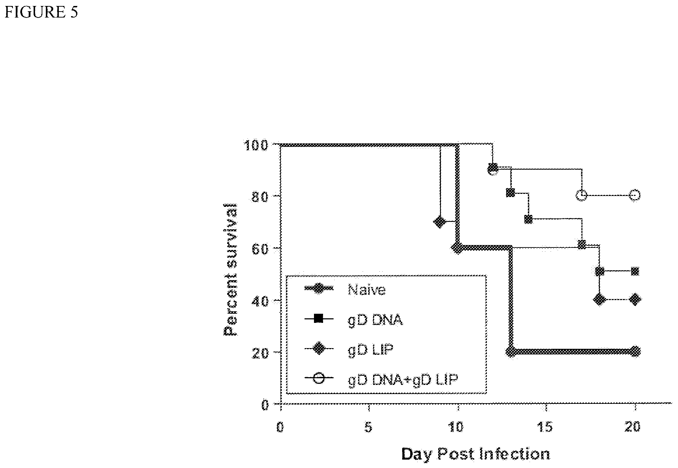

**Figure 5****.** Survival curves of immunized animals. Female Balb/c mice were either left untreated (naïve), immunized with 0.5 µg of gD DNA vaccine i.m. on days 0 and 2 (gD DNA), 15 µg of gD protein encapsulated in liposomes (gD LIP) or 0.5 µg of gD DNA i.m. on days 0 and 2 followed by gD LIP i.n. 3 weeks after the last DNA immunization (gD DNA+ gD LIP). Two weeks after the last immunization, animals were injected with progesterone and inoculated with 100 LD50 of HSV-2. Animals were examined daily for 3 weeks for clinical signs of disease. Animals with a clinical score of 4 were sacrificed. n = 5 for naïve mice; n = 10 for gD DNA, gD LIP and gD DNA+ gD LIP.

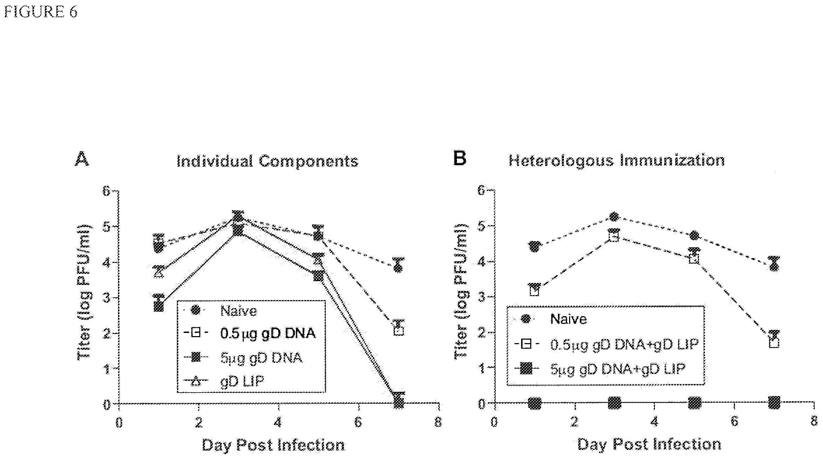

**Figure 6****.** Titration of virus isolated from vagina of vaccinated animals infected 2 weeks after immunization. Female Balb/c mice were either left untreated (naïve), immunized with 0.5 µg or 5 µg of gD DNA i.m. on days 0 and 2, 15 µg of gD protein encapsulated in liposomes (gD LIP) or 0.5 µg or 5 µg of gD DNA i.m. on days 0 and 2 followed by gD LIP i.n. 3 weeks after the last DNA immunization (gD DNA+ gD LIP). Two weeks after the last immunization, animals were injected with progesterone and inoculated with 100 LD50 of HSV-2. Vaginal washes were collected on days 1, 3, 5 and 7 post infection and titrated in Vero cells. **A.** Viral titers after vaccination with individual components. **B.** Viral titers after heterologous immunization. Titers obtained from unvaccinated mice are shown on both panels for comparison. n = 5 for naive mice, 0.5 µg gD DNA, 5 µg gD DNA, gD LIP and 0.5 µg gD DNA+ gD LIP; n = 6 for 5 µg gD DNA and n = 4 for 5 µg gD DNA+ gD LIP.

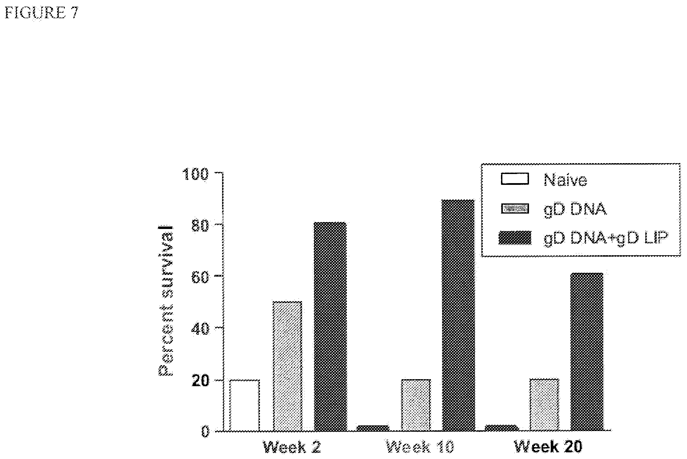

**Figure 7****.** Long-lasting protective immunity at 10 and 20 weeks post immunization. Female Balb/c mice were immunized as described in the description for Figure 5. At 10 and 20 weeks after the last immunization, animals were injected with progesterone and infected intravaginally with 100 LD50 of HSV-2. n = 5 for naive; n = 10 for gD DNA at week 2 and 20; n = 9 for gD DNA at week 10; n = 10 for all gD DNA+ gD LIP groups.

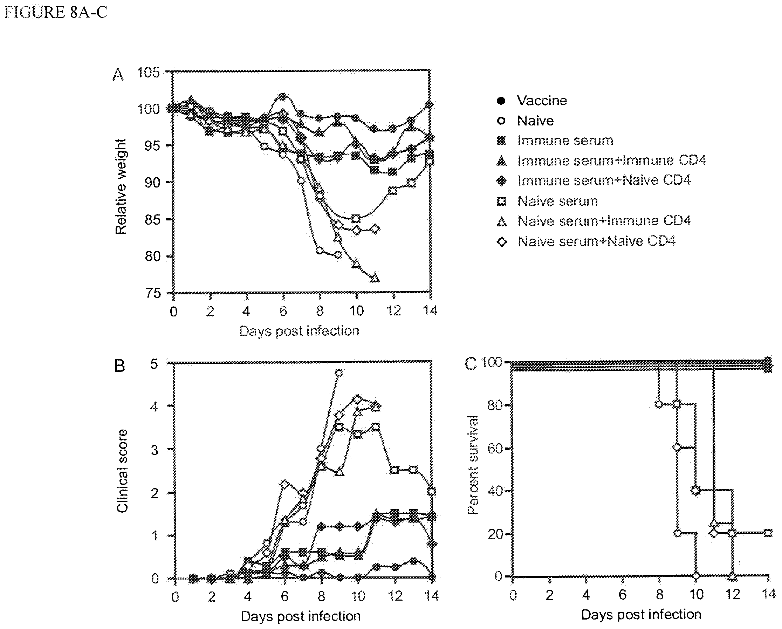

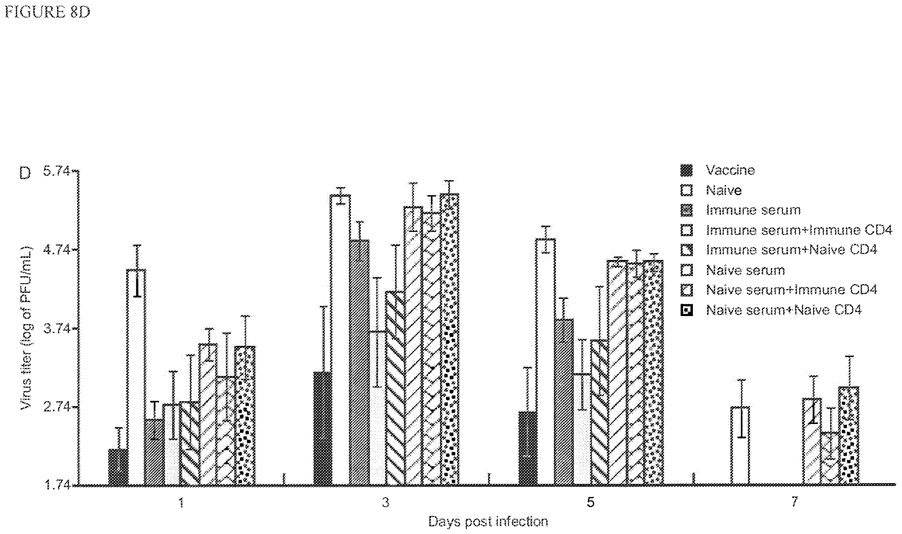

**Figure 8****.** Passive transfer of immune serum and CD4⁺ T cells protected mice from HSV-2 challenge. Mice were immunized with gD DNA *i.m.* on days 0 and 2 followed by gD LIP *i.n.* 3 weeks after the last DNA immunization ("vaccine"). Non-immunized mice ("naïve") were used as a control. Recipient, naive mice received serum from vaccinated ("immune serum") or naïve mice ("naïve serum"), CD4⁺ T cells isolated from vaccinated ("immune CD4") or naive mice ("naïve CD4"), or a combination of the two. Recipients were subsequently challenged with 2 X 10⁴ PFU of HSV-2. The approximate LD₅₀ of this HSV-2 virus stock in progesterone-treated Balb/c mice was 1.3 X 10³ PFU. Body weight **(A),** clinical score **(B),** survival **(C),** and viral load **(D)** were monitored up to 14 days post-infection.

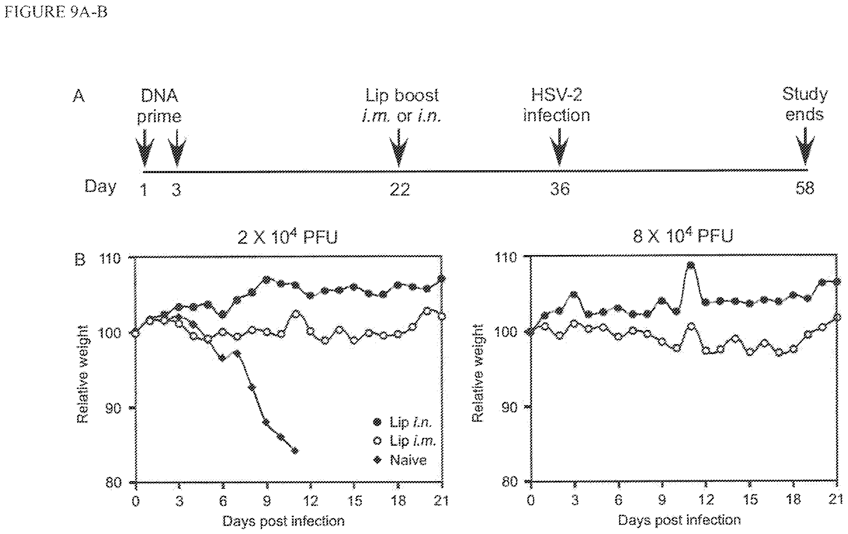

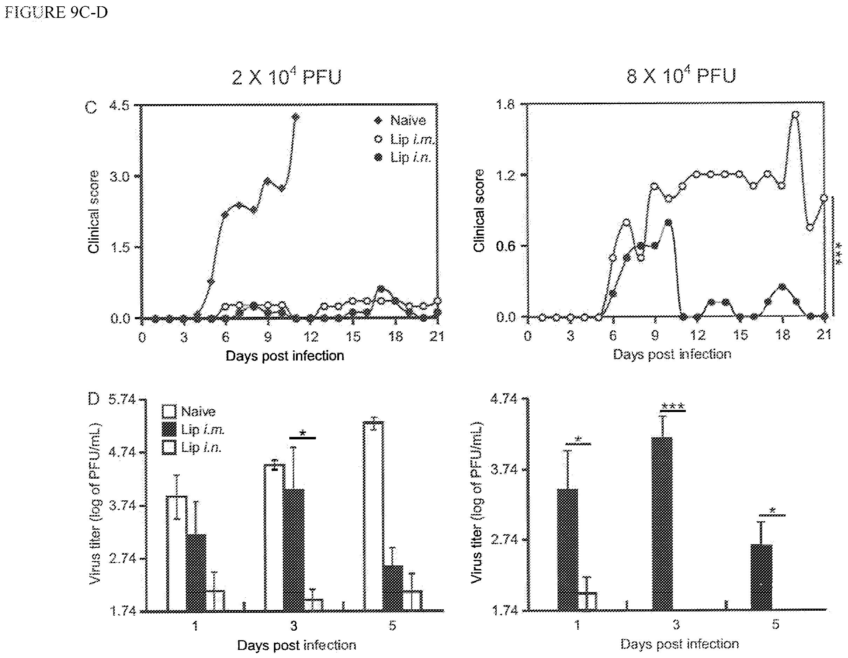

**Figure 9****.** Comparison of *i.m.* and *i.n.* boosting strategies post gD DNA priming. **A.** Balb/c mice were primed with gD DNA vaccine (*i.m*.) first and then boosted with gD Lip either *i.n.* or *i.m.* three weeks later. Two weeks following the gD Lip boost, mice were challenged with one of two doses of HSV-2 (2 X 10⁴ PFU or 8 X 10⁴ PFU). After HSV-2 challenge, the body weight **(B)** and clinical score **(C)** were recorded daily. Vaginal wash samples were collected and examined by plaque assay to titrate the virus titers **(D).**

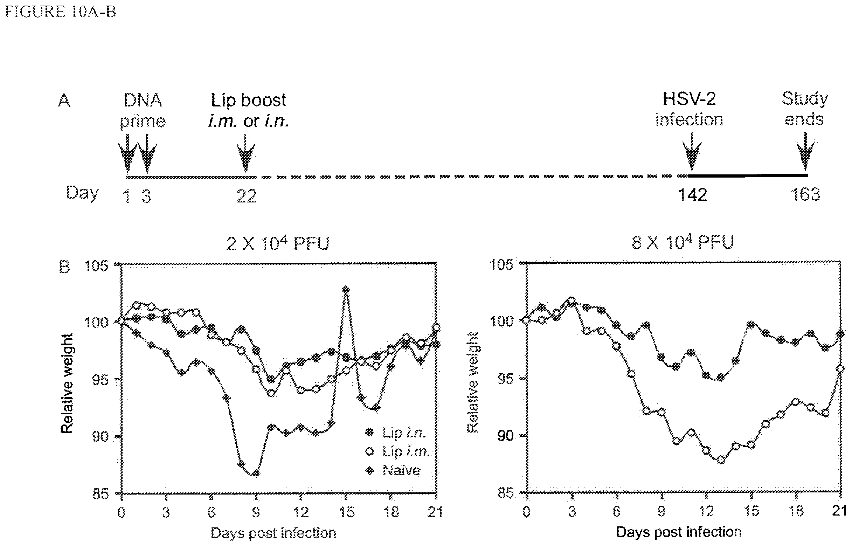

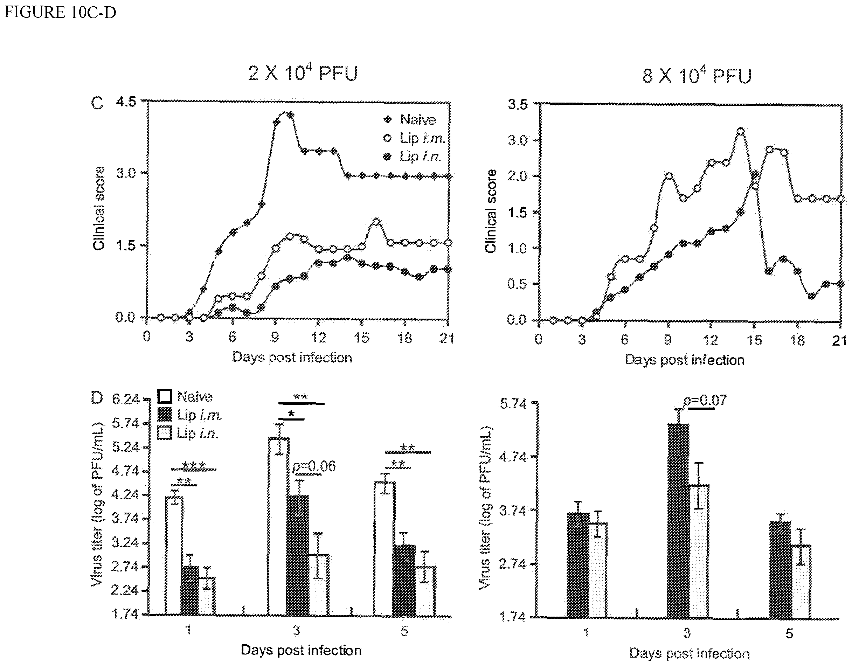

**Figure 10****.** Comparison of the role of different boosting routes (*i.n.* vs *i.m*.) on immune protection. **A.** Balb/c mice were primed with gD DNA vaccine (*i.m.*) first and then boosted with gD Lip either *i.n.* or *i.m.* three weeks later. Seventeen weeks post the gD Lip boost, all the animals were challenged with either 2 X 10⁴ or 8 X 10⁴ PFU of HSV-2. The body weight **(B)** and clinical score **(C)** were recorded daily post infection. Vaginal wash samples were collected and examined by plaque assay to titrate the virus titers **(D).**

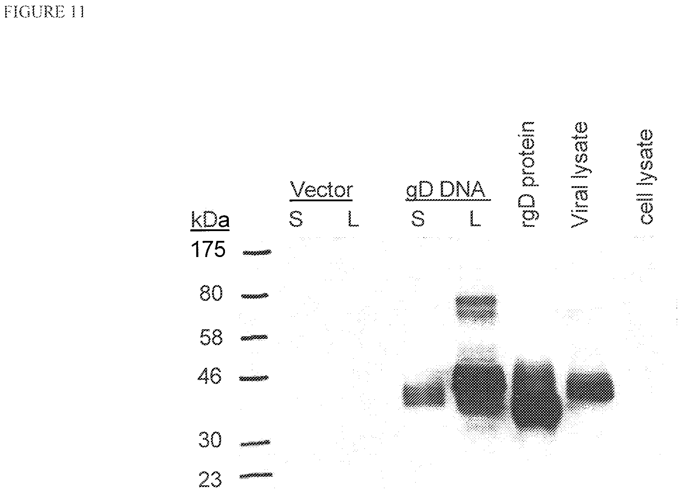

**Figure 11****.** High expression of HSV-2 gD protein by the gD DNA vaccine was detected by Western Blot. Vector S, cell supernatant of 293T cells transfected with plasmid DNA vector. Vector L, cell lysate of 293T cells transfected with plasmid DNA vector. gD DNA S, cell supernatant of 293T cells transfected with the gD DNA vaccine. gD DNA L, cell lysate of 293T cells transfected with the gD DNA vaccine. rgD protein, recombinant gD protein (extracellular domain of HSV-2 gD protein) produced in *Pichia pastoris.* Viral lysate, HSV-2 virus-infected Vero cell lysate. Cell lysate, Vero cell lysate. Anti-HSV-2 gD mouse serum reference was used as the detection antibody in Western Blot at the dilution of 1:250.

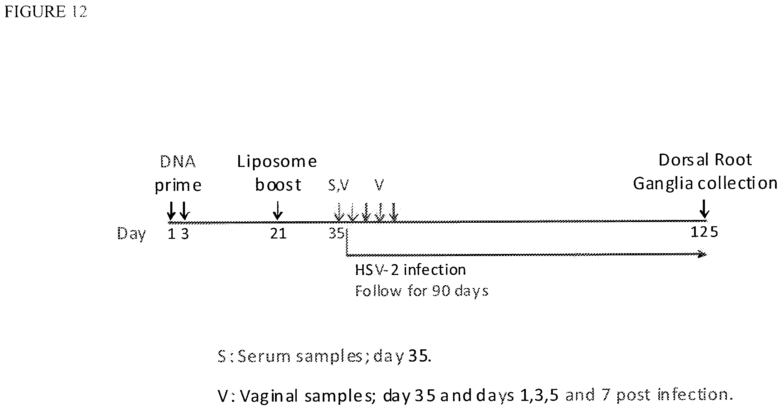

Figure 12. Timeline of vaccination and HSV-2 challenge. To test the ability of the vaccine (gD DNA, prime + gD Lip boost) to induce synergistic immune responses and protection, 10 female Dunkin Hartley guinea pigs were immunized heterologously by priming with an *i.m.* injection with 100 µg of gD DNA (given on days 0 and 2) followed by an *i.n.* boost with 30 µg of gD Lip given 3 weeks later. To evaluate the contribution from each of the vaccine components alone and together, groups of 10 guinea pigs were immunized with gD DNA followed by empty liposomes or DNA vector followed by gD Lip. Naïve guinea pigs served as a negative control. Vaginal and serum samples were collected as indicated. Post infection, animals were observed for 90 days.

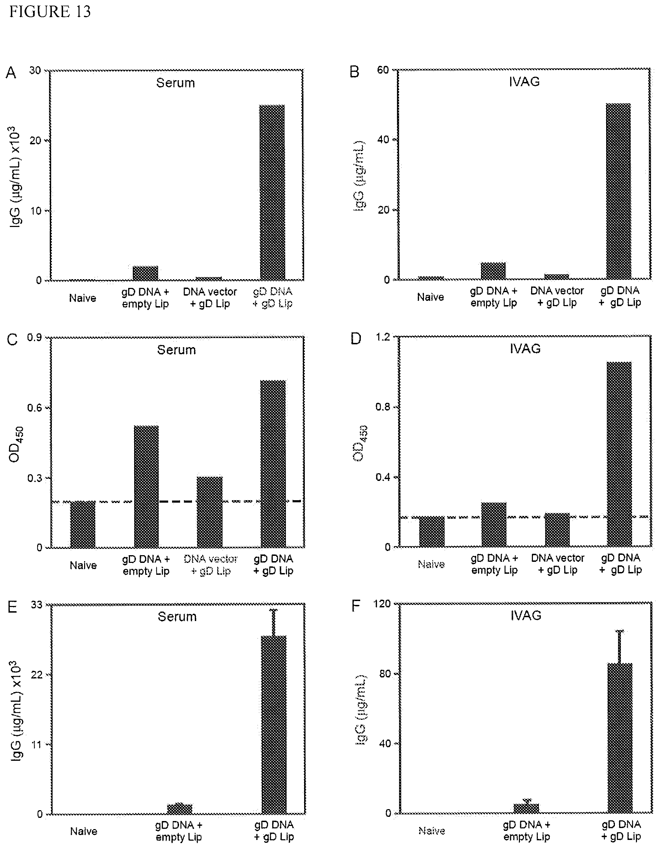

**Figure 13****.** Serum and vaginal antibody responses to heterologous immunization. Female Hartley guinea pigs were either left untreated (naïve), immunized with 100 µg of gD DNA vaccine *i.m.* on days 0 and 2 (gD DNA), 30 µg of gD protein encapsulated in negatively-charged liposomes (gD LIP), or gD DNA *i.m.* on days 0 and 2 followed by gD LIP *i.n.* 3 weeks after the last DNA immunization (gD. DNA+gD LIP). Serum and vaginal antibody responses were measured 2 weeks after the liposome immunization: gD-specific total IgG **(A and B)** and IgA **(C and D)** from pooled samples and average IgG responses for selected groups +/- SEM are shown **(E and F).** The difference in IgG levels after immunizing with gD DNA alone versus both components in combination (gD DNA+gD LIP) was highly significant (p<0.0001 for serum; p<0.001 for IVAG, n=10 per group).

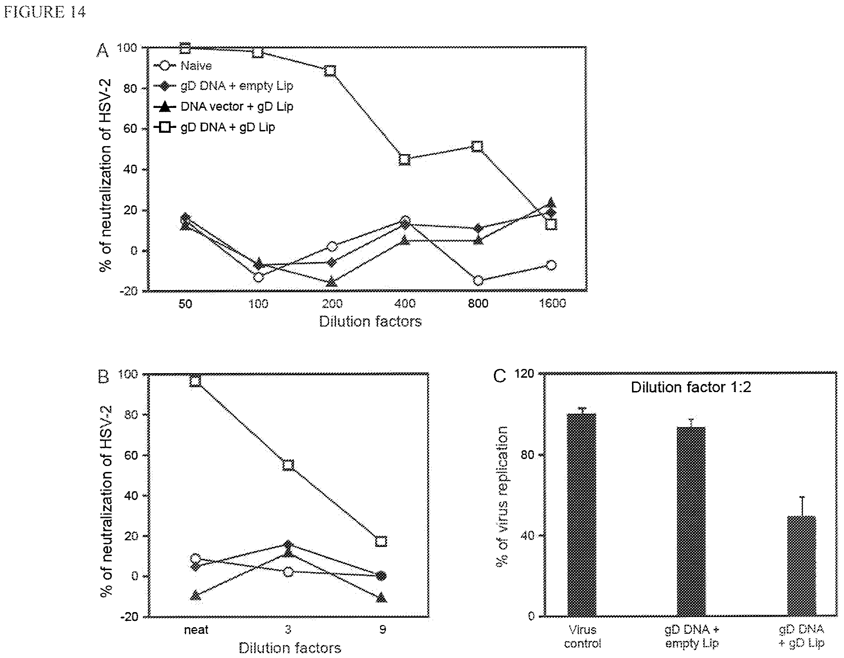

**Figure 14****.** Neutralization of HSV-2 plaque formation by serum and vaginal secretions. Female Hartley guinea pigs were either left untreated (naïve), immunized with 100 µg of gD DNA vaccine *i.m.* on days 0 and 2 followed by empty liposomes *i.n.* (gD DNA + empty LIP), DNA vector *i.m.* on days 0 and 2 followed by 30 µg of gD protein encapsulated in liposomes *i.n.* (DNA vector + gD LIP), or gD DNA *i.m.* on days 0 and 2 followed by gD LIP *i.n.* 3 weeks after the last DNA immunization (gD DNA+gD LIP). The percentage of neutralization of HSV-2 plaques is relative to virus alone with no added sample. **A.** 2-fold dilutions of serum were tested using pooled samples. **B.** 3-fold dilutions were tested using pooled vaginal samples. **C.** A 1:2 fold dilution was used for individual vaginal samples. n=10 for each group. P values are as follows: virus alone control versus gD DNA: 0.3; virus alone control versus gD DNA+gD LIP: 0.001; gD DNA versus gD DNA+gD LIP; 0.001.

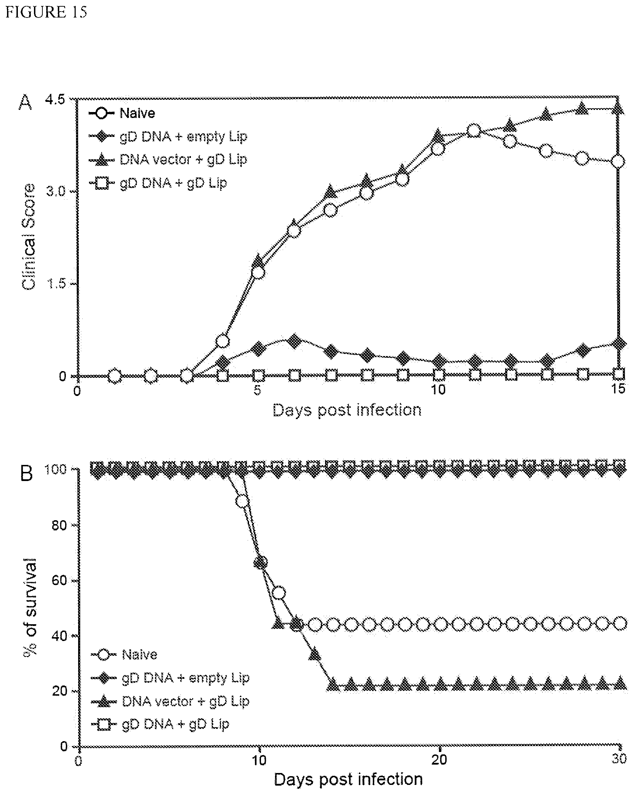

**Figure 15****.** Clinical scores Survival curves of immunized animals. Female Hartley guinea pigs were either left untreated (naive), immunized with 100 µg of gD DNA vaccine *i.m.* on days 0 and 2 followed by empty liposomes *i.n.* (gD DNA + empty LIP), DNA vector *i.m.* on days 0 and 2 followed by 30 µg of gD protein encapsulated in liposomes *i.n.* (DNA vector + gD LIP), or gD DNA *i.m.* on days 0 and 2 followed by gD LIP *i.n.* three weeks after the last DNA immunization (gD DNA+gD LIP). Two weeks after the last immunization, animals were inoculated with 2 x 10⁶ pfu of HSV-2. Animals were examined daily for 3 weeks for clinical signs of disease. Panel **A** shows average clinical scores and Panel **B** shows average % survival per group (n=9).

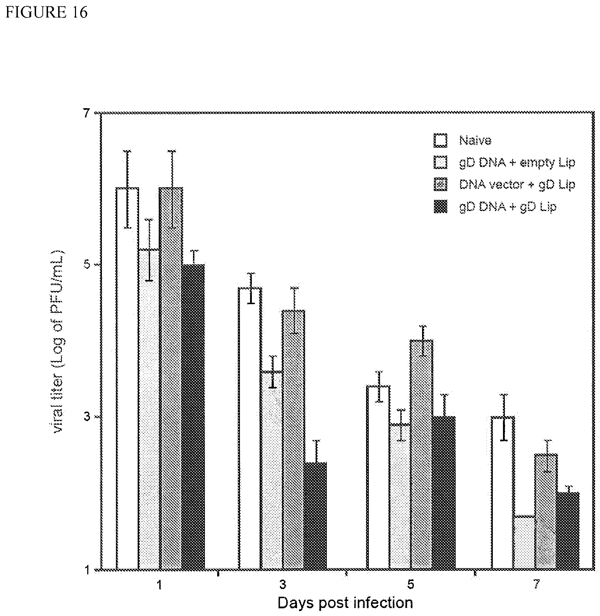

**Figure 16****.** Titration of virus isolated from vagina of vaccinated animals infected 2 weeks after immunization. Female Hartley guinea pigs were either left untreated (naïve), immunized with 100 µg of gD DNA vaccine *i.m.* on days 0 and 2 followed by empty liposomes *i.n.* (gD DNA + empty LIP), DNA vector *i.m.* on days 0 and 2 followed by 30 µg of gD protein encapsulated in liposomes *i.n.* (DNA vector+ gD LIP), or gD DNA *i.m.* on days 0 and 2 followed by gD LIP *i.n.* three weeks after the last DNA immunization (gD DNA+gD LIP). Two weeks after the last immunization animals were inoculated with 2 x 10⁶ pfu of HSV-2. n=9 for each group. The limit of detection was 1.7 log pfu/ml. Compared to unvaccinated animals, viral titers in animals vaccinated with both gD DNA+gD LIP on day 3 and day 7 were significantly lower (P=<0.01). Vaginal titers of animals on day 3 and 7 vaccinated with gD DNA alone were lower than unvaccinated guinea pigs on both days (P= 0.001). No significant difference was observed between animals vaccinated with gD LIP and unvaccinated animals.

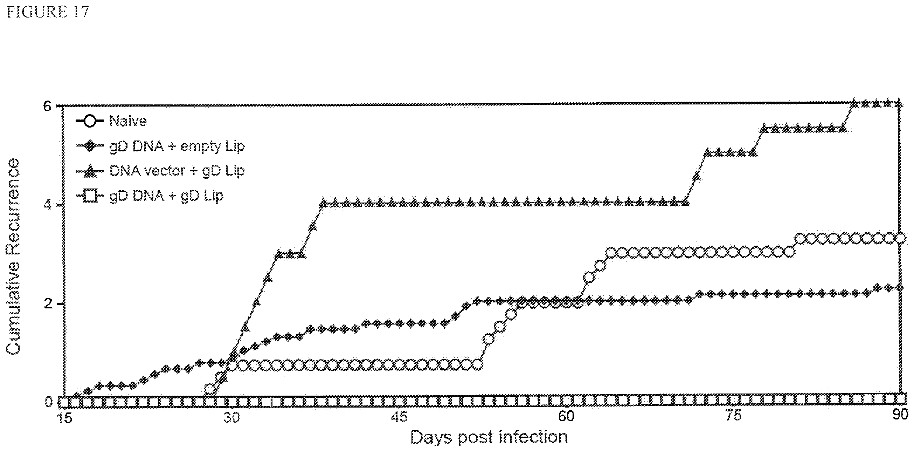

**Figure 17****.** Cumulative recurrences of HSV-2 disease per guinea pig for each immunization group, adjusted for number of days of observation. Female Hartley guinea pigs were either left untreated (naïve), immunized with 100 µg of gD DNA vaccine *i.m.* on days 0 and 2 followed by empty liposomes *i.n.* (gD DNA + empty LIP), DNA vector *i.m.* on days 0 and 2 followed by 30 µg of gD protein encapsulated in liposomes *i.n.* (DNA vector + gD LIP), or gD DNA *i.m.* on days 0 and 2 followed by gD LIP *i.n.* three weeks after the last DNA immunization (gD DNA+gD LIP). Two weeks after the last immunization, animals were inoculated with 2 x 10⁶ pfu of HSV-2. After the initial 15 day acute phase, animals were examined daily for an additional 75 days (up to day 90) for clinical signs of recurrent disease. The average total number of days that animals exhibited signs of recurrent disease is shown for each group. n=4 for naïve guinea pigs, n=9 for gD DNA, n=2 for gD LIP and n=9 for gD DNA+gD LIP. No recurrence was observed in the gD DNA+gD LIP group.

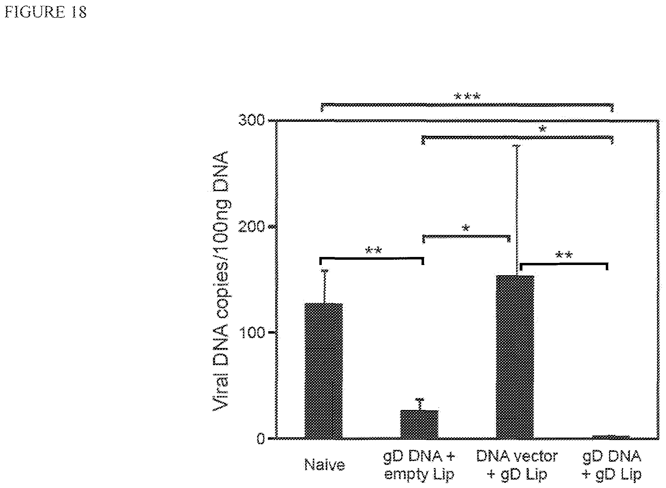

**Figure 18****.** Average latent viral genome copy number from surviving guinea pigs detected by qPCR. Animals that were not infected determined by absence of virus in vaginal swabs from day 1-7 post infection were not included in the analysis. Animals were immunized and infected as described in the description for Figure 17. Dorsal root ganglion (DRG) samples were collected from successfully infected survival animals at day 90 post infection. n=4 for naïve guinea pigs (average: 128 ± 31); n=9 for gD DNA (average: 28 +10); n=2 for gD LIP (average: 155 ± 123) and n=9 for gD DNA+gD LIP (average: 3 ± 0.6). *, p<0.05; **, p<0.01; ***, p<0.001. The gD DNA+ gD LIP group was significantly protected from establishment of HSV-2 latency.

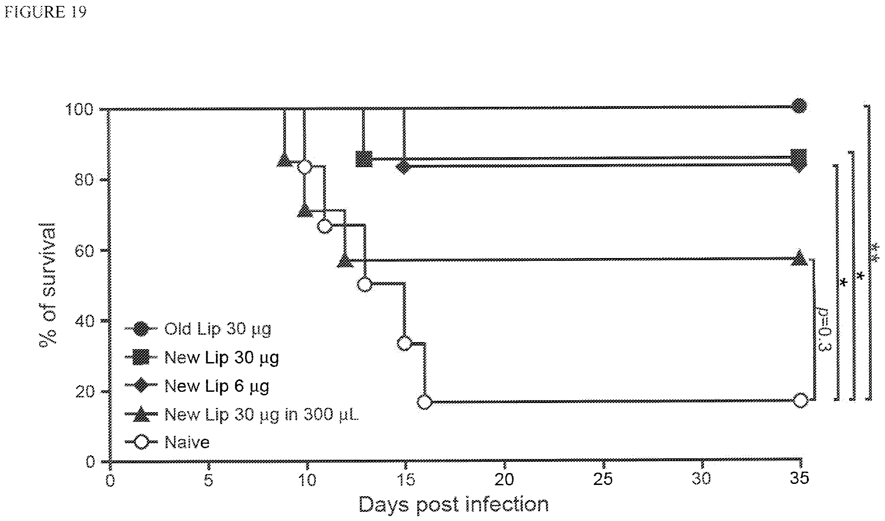

**Figure 19****.** Long-term immune protection was achieved in female guinea pigs 15 weeks post the final immunization. Female Dunkin Hartley guinea pigs were immunized heterologously by priming with an *i.m.* injection with 100 µg of gD DNA on days 0 and 2 followed by an *i.n.* boost with the indicated amount (6 or 30 µg) of gD Lip given 3 weeks later. Fifteen weeks following the gD Lip boost, all the animals were challenged with 2 x 10⁶ PFU of HSV-2. Successfully immunized and infected animals were clinically observed for 35 days post infection. The percentages of survival of each group of animals are shown. The intranasal boosting formula are: old gD Lip 30 µg in 100 µL , n=8; New gD Lip 30 µg in 100 µL, n=7; New gD Lip 6 µg in 100 µL, n=6; New gD Lip 30 µg in 300 µL, n=7; Naive, n=6. *, p<0.05; **, p<0.01.

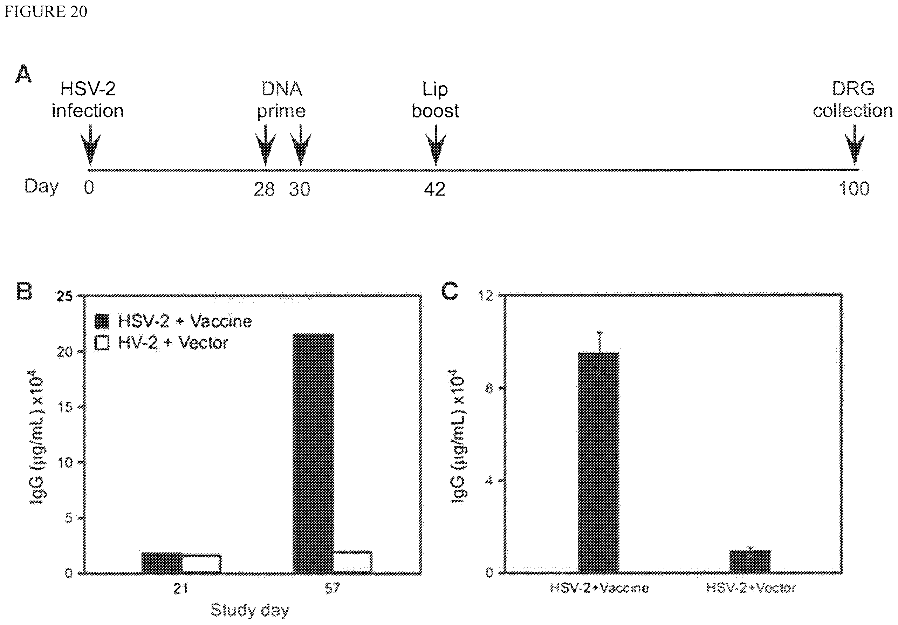

**Figure 20****. A.** Schematic showing the therapeutic vaccination regimen in HSV-2 infected guinea pigs. Four weeks post intravaginal infection with HSV-2, the DNA prime was injected intramuscularly over two days. Two weeks later the intranasal liposome boost was administered. The guinea pigs were monitored daily for an additional 60 days for signs of recurrent disease. Serum samples were collected on days 21 and 57; vaginal swab samples were collected on days 2, 4, 21 and 57; dorsal root ganglia were harvested at the end of the study to determine the latent HSV-2 DNA copy numbers. **B and C.** Female Hartley guinea pigs were either treated with gD DNA + gD liposome vaccine or DNA vector + empty liposome vehicle four weeks post infection with HSV-2. Serum anti-HSV-2 gD IgG antibody responses were measured one week before the DNA prime (day 21) and 2 weeks after the liposome boost (day 57). Anti-gD-specific IgG from pooled samples **(B)** and average IgG responses for individuals on day 57 +/- SEM are shown **(C).** n= 15 for both vaccine and vehicle groups.

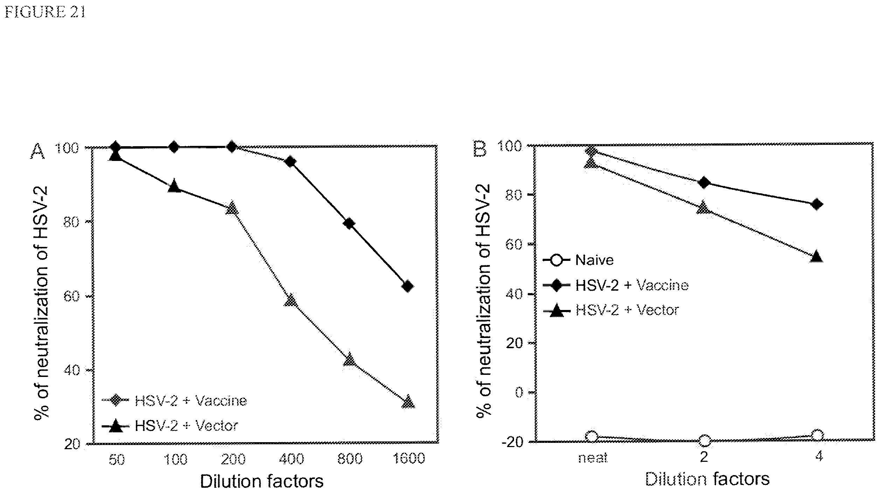

**Figure 21****.** Vaccine treatment induced higher neutralization antibody responses in guinea pigs exposed to HSV-2 infection. Animals were infected with HSV-2 and then treated with vaccine (gD DNA + gD LIP) or control vehicle (DNA vector + empty liposomes). Two weeks after the last immunization (study day 57), serum and vaginal swab samples were collected from all the animals and tested in a virus neutralization assay. The neutralization antibody activity of the pooled serum sample **(A)** and the pooled vaginal samples **(B)** are shown.

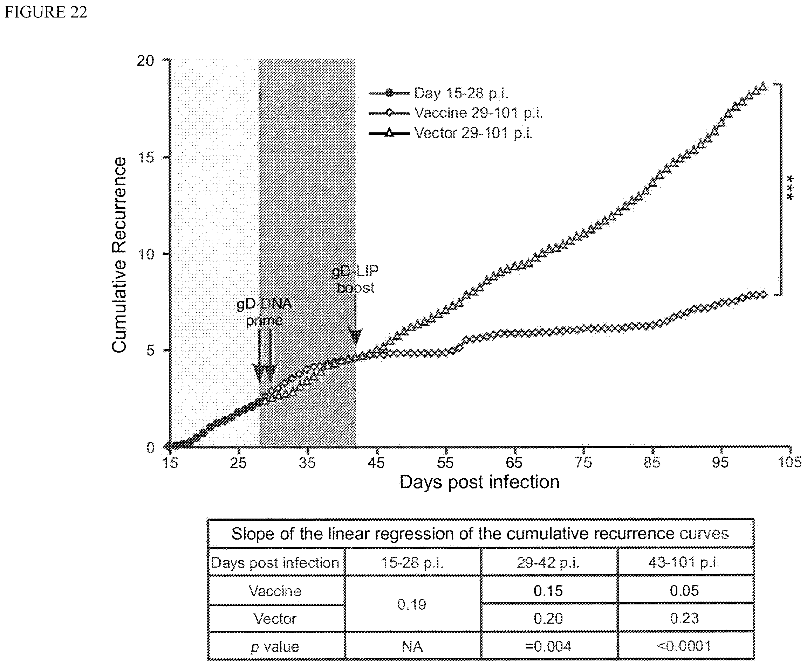

**Figure 22****.** Cumulative recurrence per animal per group in vaccine-treated and vector-treated guinea pigs following acute HSV-2 infection. After immunization of infected guinea pigs, the animals were observed for 60 days for evidence of spontaneous recurrent herpatic lesions. Recurrent episodes were enumerated as cumulative recurrences (appearance of lesions) per guinea pig for each group, adjusted for the number of days the recurrences were observed. Light grey is for the pre-treatment period (day 15-28 post infection), and dark grey for vaccine treatment period (day 29-42 post infection). The table lists the statistical differences in slope of linear regression for selected periods of days post infection.

### DETAILED DESCRIPTION OF THE INVENTION

Many different HSV-2 immunization strategies have been developed and evaluated, including the use of whole inactivated virus, live attenuated virus, live replication defective virus, subunit vaccines and DNA vaccines. However, a safe and effective vaccine for HSV-2 is still not available. The present invention is based, in part, on the discovery of a new HSV-2 vaccine that elicits protective and therapeutic immunity against HSV-2. As described in detail herein, the inventors have developed a heterologous immunization protocol comprised of a DNA prime given intramuscularly and a liposome-encapsulated boost delivered mucosally (e.g. intranasally). The vaccine induces clear synergistic responses comprised of humoral, T cell and mucosal immunity. Specifically, the vaccine stimulates high titers of serum antibodies, a Th1-biased immune response and potent mucosal immunity. The immune responses elicited by the methods of the present invention can protect immunized animals from challenges by live HSV-2 virus. The methods of the invention can also be employed to induce therapeutic immune responses in animals (e.g. humans) with HSV-2 infections to prevent or reduce recurrences of herpes disease.

Thus, the present invention provides a priming preparation and a boosting preparation for use in a method for eliciting a protective immune response against HSV-2 in a mammal wherein the method comprises administering to the animal a priming preparation comprising a vector encoding an HSV-2 antigen under the control of a promoter, wherein the antigen is a full-length HSV-2-gD glycoprotein, and wherein the sequence encoding the gD glycoprotein is codon-optimized for expression in mammalian cells and a boosting preparation comprising an extracellular domain of the gD glycoprotein encapsulated in liposomes wherein in the method, the priming preparation is administered intramuscularly and the boosting preparation is administered mucosally, such as intranasally, orally, intravaginally, rectally, sublingually, buccally or via inhalation. In one particular embodiment, the boosting prepartion is administered intranasally. In such embodiments, the method induces high titers of serum and vaginal antibodies with high neutralizing activities, a Th1 type biased response, and potent protective immunity at the vaginal cavity, the portal of entry for the HSV-2 virus. In another embodiment, priming with HSV-2 antigen-DNA vaccine intramuscularly and boosting with HSV-2 antigen-liposomes intranasally significantly reduces the viral vaginal load, enhances viral clearance during the acute phase, completely protects immunized animals from developing clinical signs of disease up to 90 days post HSV-2 viral challenge, and prevents establishment of HSV-2 viral latency in dorsal root ganglia of the animal. Delivery of the boost by the intranasal route was established as an important determinant for the generation of local mucosal immunity and was associated with a quicker virus clearance from the vaginal site and a more efficient protection against HSV-2 induced disease.

As used herein, a "protective immune response" or "protective immunity" refers to immunity or eliciting an immune response against an infectious agent, which is exhibited by a vertebrate (e.g., a human), that prevents or protects against infection. A protective immune response that prevents or protects against the appearance of disease symptoms will reduce or stop the spread of HSV-2 in a population by reducing viral shedding. In some embodiments, the protective immunity induced by the vaccine of the present invention is a sterilizing immunity. "Sterilizing immunity" is an immune response that completely eliminates or prevents an infection or is rapidly cleared, leaving no detectable trace.

Also described is a method for modulating immune responses such that a desired immune response biased towards a T helper type 1 (Th1) response may be elicited in an animal. The method comprises administering intramuscularly to the animal a priming preparation comprising a nucleic acid sequence encoding an antigen, such as an HSV-2 antigen, and subsequently administering to the animal a boosting preparation comprising the antigen encapsulated in liposomes. "Biased towards" refers to the situation where the observed immune response is closer to a Th1 or T helper type 2 (Th2) response compared to before immunization. In certain embodiments, immunization will completely switch a Th2 response to a Th1 response. In other embodiments, immunization may not completely switch a Th2 response to a Th1 response, but instead, results in a mixed or balanced response or a weaker Th2 response.

Most immune responses are regulated by T lymphocytes, which initiate and shape the nature of the response. As immune responses mature, CD4⁺ T lymphocytes can become polarized towards T helper type 1 (Th1) or T helper type 2 (Th2) immune responses. The hallmark of Th1 and Th2-type responses is the predominant pattern of cytokines that are present. Th1 responses are characterized by high levels of IFN-γ and low levels of IL-4 and IL-10, while Th2 responses are characterized by low levels of IFN-γ and high levels of IL-4 and IL-10. These cytokines play an important role in determining the functional capabilities of the T cells. Th2-type responses lead to the preferred production of antibodies of the IgG1 subclass, with little or no generation of CTLs. Th1-type responses lead to the preferred production of antibodies of the IgG2a subclass and induction of CTLs that can effectively kill cells infected with viruses or other organisms.

Table 1 below summarizes the immunological characteristics of Th1 and Th2 polarized immune responses. Th1 polarized responses are typically generated during infections with viruses or bacteria. In contrast, Th2 polarized responses are often observed in parasitic infections, in allergic responses, and by conventional alum-based intramuscularly delivered protein vaccines that are used in humans. Genetics can also determine the type of immune responses generated. For example, Th1 responses predominate in the C57BL/6 strain of mouse, while Th2 responses predominate in the Balb/c strain of mouse. Immune responses may also consist of both Th1 and Th2 components, affording protection by both humoral and cell mediated arms of the immune response. Direct determination of the frequencies of cytokine producing cells is accomplished by the use of ELISPOT (Enzyme Linked Immunosorbent SPOT assays) or by immunofluorescence staining to reveal intracellular cytokine production. Serum IgG1:IgG2a ratios are also widely accepted and followed criteria to determine T helper types (Table 1). An IgG1 to IgG2a ratio for balanced Th1 and Th2 response would be between 0.5 and 2.0.

**Table 1. A Characteristics of Th1 and Th2 polarized T cell responses.**

| Immune Responses | Th1-type immune responses | Th2-type immune responses |
|---|---|---|
| Humoral immunity | Serum IgG1/IgG2a < 0.5 | Serum IgG1/IgG2a > 2.0 |
| T cell cytokine secretion | ↑ IFN-γ; ↓ IL-10 and IL-4 | ↑ IL-10 and IL-4; ↓ IFN-γ |
| CTL | High | Low or absent |
| Prototypical mouse strains | C57BL/6 | Balb/c |

As used herein, "T helper type 1 response" and "Th1 response" are used interchangeably to refer to a range of host animal responses including one or more, usually all the characteristics listed in the middle column of Table 1 above. These characteristics include a ratio of IgG1:IgG2a of no greater than 0.5; increased IFN-γ (and other Th1 cytokines) secretion by T helper 1 cells and decreased IL-10 and IL4 (and other Th2 cytokines) secretion by T helper 2 cells; and high CTL activity.

Similarly, as used herein, "T helper type 2 response" and "Th2 response" are used interchangeably to refer to a range of host animal responses including one or more, usually all the characteristics listed in the right column of Table 1 above. These characteristics include a ration of IgG1:IgG2a of no less than 2.0; decreased IFN-γ (and other Th1 cytokines) secretion by T helper 1 cells and increased IL-10 and IL-4 (and other Th2 cytokines) secretion by T helper 2 cells; and low or absent CTL activity.

The disclosure additionally provides a method to develop effective vaccines and vaccination protocols to specifically target the most common entry portal for microorganisms, the mucosal surfaces of the body. The disclosure provides that vaccines could be made more effective by encapsulating them into liposomes with a defined composition/size, and by delivering them directly to mucosal surfaces. By combining liposome encapsulated vaccines with appropriate immunization regimens, immune responses can be tailored to provide more effective and specific vaccines. In certain cases, the ideal vaccine generates a balanced or T helper 1 (Th1)-biased immune response that also includes robust antibody responses, CTL generation and Th1-type cytokine production, and local immunity at mucosal sites. In other cases, it may be possible to tailor the immune response to generate a T helper 2 (Th2)-biased immune response, which may be beneficial in preventing rejection in graft hosts and in protecting against certain parasitic infections.

Also described is a kit for eliciting a protective immune response against HSV-2 in an animal (*e.g*. mammal). In one embodiment, the kit comprises a first immunizing component comprising a nucleic acid sequence encoding an HSV-2 antigen, a second immunizing component comprising the HSV-2 antigen encapsulated in liposomes, and an instruction for a user to administer to the animal the first immunizing component followed with administering the second immunizing component to elicit the protective immune response in the animal. In some cases, the first immunizing component is formulated for intramuscular administration and the second immunizing component is formulated for mucosal administration, for instance intranasally, orally, intravaginally, rectally, sublingually, buccally or via inhalation. In certain cases, the second imunizing component is formulated for intranasal administration. In another case, the kits further comprise a delivery device for adminstering the first immunizing component, the second immunizing component, or both. The delivery device can be any of the delivery devices described *infra,* including droppers, swabs, aerosolizers, insufflators, nebulizers, inhalers, syringes equipped with needles or autoinjectors.

The methods of the invention are useful for therapeutic vaccination. The goal of therapeutic vaccination is to target immune responses in an infected individual to eradicate cells already infected with virus or prevent the latent viruses from reactivation and trafficking back to the original infection sites. As used herein, "therapeutic immunity" or a "therapeutic immune response" refers to immunity or eliciting an immune response against an infectious agent that ameliorates or eliminates an infection or reduces at least one symptom thereof. Specifically, induction of a therapeutic immune response from administration of the vaccine is evident by elimination or reduction of the presence of one or more symptoms of disease induced by the infectious agent or a reduction in the duration or severity of such symptoms. For instance, in one embodiment, therapeutic immunity against HSV-2 refers to immunity that reduces the severity or duration of a HSV-2 infection. Therapeutic immunity, in some embodiments, is manifested by elimination or reduction of the presence of one or more symptoms of HSV-2-induced disease. Clinical symptoms of HSV-2-induced disease include blister-like sores or ulcerations around the genital and/or rectal areas, a rash or small bumps on the skin in the genital and/or rectal areas, painful urination, vaginal fluid discharge, and flu-like symptoms (e.g., fever and swollen lymph glands in the groin).

Also described is a method for treating an HSV-2 infection in an animal (*e.g*. mammal, human) comprising administering to the animal a priming preparation comprising a nucleic acid encoding an HSV-2 antigen, and a boosting preparation comprising the antigen encapsulated in liposomes. In the invention, the priming preparation is administered intramuscularly and the boosting preparation is administered mucosally. Mucosal routes of administration include, but are not limited to, intranasal, oral, vaginal, rectal, sublingual, buccal, or via inhalation to the lungs. In one particular embodiment, the boosting preparation is administered intranasally. Preferably, one or more symptoms of HSV-2 infection is alleviated, ameliorated, reduced, or cured following administration of the heterologous prime and boost HSV-2 vaccine. For instance, the number, severity or frequency of genital lesions is significantly reduced in an immunized animal as compared to an untreated animal. In one embodiment, the recurrence of herpatic lesions is reduced in the immunized animal as compared to an untreated animal. In another embodiment, viral shedding from the genital tract is reduced in the immunized animal as compared to an untreated animal.

Other viruses that establish long-term infections, which can be treated with the methods and kits of the disclosure, include human papilloma virus (HPV), the hepatitis B and C viruses (HBV, HCV), and human immunodeficiency virus (HIV). The development of therapeutic vaccines for viruses has focused on the activation of CTL to recognize and destroy infected cells and/or controlling the virus spread. Applicants have shown the methods of the invention are effective in enhancing cellular immune responses, making them suitable for providing therapeutic vaccination. The effectiveness of the methods may be further enhanced by inclusion of cytokine adjuvants and CpG motifs that have been shown to be particularly promising in the development of anti-cancer vaccines (Belardelli et al., Cancer Res. 64:6827-6830 (2004)).

In one aspect, the present invention provides methods, for effectively eliciting immune responses, such as protective and therapeutic immune responses in mammals (*e.g.* humans), against HSV-2 antigens. One salient feature of the invention relates to the use of liposome-encapsulated protein antigens delivered mucosally (*e.g*. intranasally (IN)) or intramuscularly (IM) to the host animal.

One component of the methods of the present invention is the use of a "priming" immunization, comprising the initial administration of the antigen to a mammal, especially a human patient, in preparation for subsequent administration(s) of the same antigen. Specifically, the term "priming," as used herein, refers to a first immunization using an antigen which induces an immune response to the desired antigen and recalls a higher level of immune response to the desired antigen upon subsequent reimmunization with the same antigen when administered in the context of the same or a different vaccine delivery system.

Another component of the methods of the present invention is the use of a "boosting immunization," or a "boost," which means the administration of a composition delivering the same antigen as encoded in the priming immunization. A boost is sometimes referred to as an anamnestic response, *i.e.* an immune response in a previously sensitized animal. Multiple boosts can be administered, utilizing the same or differing amounts for each boost. A boosting that uses an antigen-delivery system different from the priming can be referred to as a "heterologous boost," whereas a boosting that uses the same antigen-delivery system as the priming can be referred to as a "homologous boost."

As an alternative to sequential administration as described above, the priming preparation and the boosting preparation may be administered simultaneously, *i.e.* at substantially the same time. The methods of the invention lead to potent synergistic effects between the priming immunization and the boosting immunization in terms of immune responses the methods are able to elicit in an animal. As a result, the methods of the invention enable one to elicit a desired level of immune response, where each of the priming preparation and the boosting preparation, when administered alone to the animal, is insufficient to accomplish the desired level of immune response. In some embodiments, heterologous immunization with intranasal boost generates a protective immune response characterized by an increase in the serum level of antigen-specific IgG and IgA compared to the serum level of antigen-specific IgG and IgA prior to the administration of the boosting preparation. In other embodiments, heterologous immunization with intranasal boost generates a protective immune response comprising a secretory IgA response and/or a mucosal IgG response. In certain embodiments, heterologous immunization with intranasal boost generates mucosal cytotoxic T lymphocytes (CTL), and provides complete protection against live pathogen challenge, such as challenges by live HSV-2 virus as shown by Applicants in the illustrative examples. In other embodiments, the heterologous immunization with intranasal boost induces a protective immune response comprising neutralizing antibodies in the serum and mucosal secretions, such as vaginal secretions. In still other embodiments, the heterologous immunization with intranasal boost induces a protective immune response that is biased towards a Th1 type immune response. In certain embodiments, the heterologous immunization with intranasal boost reduces viral load at mucosal sites (*e.g*. vagina), enhances viral clearance (*e.g*., greater than 50%, greater than 75%, greater than 85%, greater than 90%, greater than 95% viral clearance) and/or completely eliminates infective virus as compared to animals immunized with only the priming or boosting component alone, and prevents establishment of viral latency, such as the integration of HSV-2 viral genomes within the cells of the dorsal root ganglia of host animals.

The effects of immune response in the host animal, including humoral and cellular immune responses, can be assessed by various assays known in the art. Humoral immune response includes total antigen-specific antibody (immunoglobulins, *i.e.,* Ig) titers in serum or at mucosal surfaces; titers of HSV-2 antigen-specific antibodies in serum or at mucosal surfaces; titers of specific antibody isotypes and/or sub-types including IgG, IgA, IgG1, and IgG2a; ratio of IgG1 and IgG2a. Antigen-specific antibody titers can be measured by routine methods known in the art, such as ELISA assays. The cellular immune response includes cytotoxic T cell (CTL) phenotype and activity. Cellular immune response also includes secretion of cytokines characteristic of Th1 responses including IFN-γ, and secretion of cytokines characteristic of Th2 response including IL-10 and IL-4. The cytokines are detected directly by cytokine ELISPOT and/or ELISA assays (*i.e.,* IFN-γ, IL-10 and/or IL-4) and inferred from IgG1:IgG2a ratios (e.g., Th1 versus Th2 response). Neutralization activity of serum and mucosal antibodies can be measured by various methods including complement-dependent neutralization assays as described in the Examples. The degree of HSV-2 viral latency in the nervous system can be assessed by, for example, quantitative real-time PCR (qPCR) analysis of dorsal root ganglia cells. The number of viral copies detected correlates to HSV-2 viral integration in the host, neuronal cell.

Each of the priming preparation and the boosting preparation may be administered by any one of the following routes: subcutaneously, intramuscularly, intradermally and mucosally, including using electronic/mechanical devices and/or methods such as electroporation. Exemplary routes of mucosal administration include, but are not limited to, intranasal, oral, vaginal, rectal, sublingual, buccal, or via inhalation. The priming preparation and the boosting preparation may be administered by the same route, or by different routes. In certain preferred embodiments, the priming preparation is administered intramuscularly and the boosting preparation is administered intranasally, which results in not only robust systemic immune responses but also the induction of local immunity at mucosal sites. In one particular embodiment, priming with HSV-2 antigen-DNA vaccine intramuscularly and boosting with HSV-2 antigen-liposomes intranasally induced potent synergistic neutralizing antibody responses in the serum and vaginal secretions, activated CTLs and Th1-type cytokine profiles, and potent protective immunity in the vaginal cavity, characterized by reduced viral load, enhanced and/or complete viral clearance. Delivery of the boost by the intranasal route was found to be an important determinant for the generation of local mucosal immunity and was associated with a quicker virus clearance from the vaginal site and a more efficient protection against HSV-2 induced disease.

The disclosure also provides devices for dispensing the priming and boosting preparations described herein for use in the methods of the invention. For example, for embodiments in which the boosting preparation is delivered intranasally, dispensing devices for intranasal formulations may, for example, take the form of an aerosol delivery system, and may be arranged to dispense only a single dose, or a multiplicity of doses. Such a device would deliver a metered dose of the boosting preparation to the nasal passage. Other examples of appropriate devices include, but are not limited to, droppers, swabs, aerosolizers (*e.g*. VaxINator™), insufflators (*e.g*. Valois Monopowder Nasal Administration Device, single dose Bespak UniDose DP dry powder intranasal delivery device), nebulizers, and inhalers. The devices may deliver the boosting preparation by passive means requiring the subject to inhale the formulation into the nasal cavity. Alternatively, the device may actively deliver the boosting preparation by pumping or spraying a dose into the nasal cavity. The boosting preparation may be delivered into one or both nostrils by one or more such devices. Administration could include two devices per subject (one device per nostril).

Different intervals between the priming preparation and the boosting preparation may be used. The boost preparation can be administered 2-8 weeks, preferably 4-6 weeks, or more preferably about 2-4 weeks apart from the previous administration (initial or a prior boost). Typically, one boost administered 2-4 weeks after the initial priming administration is sufficient. In one embodiment, the boost is administered 2 weeks after the priming preparation. In another embodiment, the boost is administered 3 weeks after the priming preparation. The invention contemplates that either the priming preparation or the boosting preparation, or both, may be administered to the animal one or more times. For instance, the priming preparation may be administered over the course of two or three days. In one embodiment, the priming comprises one or two administrations separated by about 3-6 weeks, and said boosting preparation is administered about 3 to 10 weeks following the last priming. In another embodiment, the priming comprises one or two administrations separated by 3 days, and said boosting preparation is administered 3 weeks following the last priming. In a certain embodiment, the priming comprises two administrations separated by a day (*e.g*. prime preparation administered on day 1 and day 3) and the boosting preparation is administered 2-3 weeks following the last priming.

The boost administration(s) and the initial administration(s) may use the same or different amounts of antigen-liposome preparations, and the boost and the initial administrations can be administered via the same or different routes. The initial administration(s) and the boost administration(s) may use different amounts of DNA antigen delivered according to different schedules, and different amounts of antigen-liposome preparations delivered.

As used herein, the terms "antigen" or "immunogen", used interchangeably, are intended to encompass all peptide or protein sequences which are capable of inducing an immune response within the animal concerned. The terms "antigen" or "immunogen" encompass peptide or protein analogs of known or wild-type antigens, which analogs may be more soluble or more stable than wild type antigen, and which may also contain mutations or modifications rendering the antigen more immunologically active or optimized for expression in certain cell types *(i.e.* humanized codon usage). An antigen may also be a peptide in which particular amino acid substitutions have been made to a naturally-occurring antigen that alter protein structure, a portion of the naturally-occurring antigen including known protective epitopes *(i.e.* CTL epitopes), or a synthetically derived string of known epitopes that may or may not be limited to one pathogen (multivalent vaccine).

Further peptides or proteins that have sequences homologous with a desired antigen's amino acid sequence, where the homologous antigen induces an immune response to the respective pathogen, are also useful. Genes that are homologous to the desired antigen-encoding sequence should be construed to be included in the instant invention provided they encode a protein or polypeptide having a biological activity substantially similar to that of the desired antigen.

Analogs of the antigens described herein can differ from naturally occurring proteins or peptides by conservative amino acid, sequence differences or through modifications that do not affect sequence, or by both. For example, conservative amino acid changes may be made, which although they alter the primary sequence of the protein or peptide, do not normally alter its function. Modifications (which do not normally alter primary sequence) include *in vivo,* or *in vitro* chemical derivatization of polypeptides, *e.g.,* acetylation, or carboxylation. Also included as antigens are proteins modified by glycosylation, *e.g.*, those made by modifying the glycosylation patterns of a polypeptide during its synthesis and processing or in further processing steps; *e.g.,* by exposing the polypeptide to enzymes which affect glycosylation, *e.g.,* mammalian glycosylating or deglycosylating enzymes. Also included as antigens according to this invention are sequences which have phosphorylated amino acid residues, *e.g*., phosphotyrosine, phosphoserine, or phosphothreonine. Also included as antigens are polypeptides that have been modified using ordinary molecular biological techniques so as to improve their resistance to proteolytic degradation or to optimize solubility properties. Analogs of such polypeptides include those containing residues other than naturally occurring L-amino acids, *e.g*., D-amino acids or non-naturally occurring synthetic amino acids. The antigens of the invention are not limited to products of any of the specific exemplary processes listed herein.

An antigen can be a full-length or a truncated antigen, an immunogenic fragment thereof, or an epitope derived from the antigen. In certain embodiments, the pathogen-specific antigen in the boosting preparation may be in the form of an attenuated or killed pathogen. Effective antigens also include surface antigens of these pathogens.

The term "epitope" as used herein refers to a sequence of at least about 3 to 5, preferably about 5 to 10 or 15, and not more than about 1,000 amino acids (or any integer therebetween), which define a sequence that by itself or as part of a larger sequence, binds to an antibody generated in response to such sequence or stimulates a cellular immune response. The term "epitope" encompasses sequences identical to the native sequence, as well as modifications to the native sequence, such as deletions, additions and substitutions (generally conservative in nature). The antigens used in the invention may comprise only a single epitope, such as, for example, a single CTL epitope.

The antigens encoded by the nucleic acids in the priming preparation and the antigens in the boosting preparation preferably have overlapping epitopes. In certain embodiments, the two antigens may be identical to each other. Alternatively, the two antigens may have overlapping but different set of epitopes. By way of an illustrating example, in a vaccination protocol for HSV-2, a DNA encoding an HSV-2 glycoprotein may be used in the priming preparation, and the boosting preparation may be inactivated HSV-2. By way of another illustrating example, the priming preparation may be a vector encoding an HSV-2 antigen, and the boosting preparation may comprise a protein form of the antigen, or vice versa.

Suitable HSV-2 antigens include, but are not limited to, gH, gL gM gB gC gK gE or gD glycoproteins or derivatives thereof or Immediate Early proteins such as ICP27, ICP 47, ICP4, ICP36. The gD glycoprotein may be the full-length form of the protein (*e.g.* SEQ ID NO: 3) or a truncated form, such as the extracellular domain of gD, or immunogenic fragments thereof. The truncated forms of HSV-2 glycoproteins (e.g. gD glycoprotein) can include truncated forms that are secreted by cells expressing the glycoprotein. For instance, in some embodiments, the antigen is the extracellular domain of gD consisting of amino acids 1-314 of gD protein. In other embodiments, the antigen is the extracellular domain of gD comprising the amino acid sequence of SEQ ID NO: 2. Variants of the full-length or truncated forms of gD can also be used. For instance, in one embodiment, the gD glycoprotein comprises a sequence of SEQ ID NO: 4, SEQ ID NO: 5, SEQ ID NO: 6, or SEQ ID NO: 7. The full-length or truncated forms of HSV-2 glycoproteins (*e.g*. gD glycoprotein) may comprise signal peptides and/or purification tags (*e.g*. histidine or c-myc tags). The sequence encoding the antigen is optimized for expression in mammalian cells, such as human cells. For instance, for optimization in human cells, cis-acting motifs, such as internal TATA-boxes, chi-sites, ribosomal entry sites, AT-rich or GC-rich sequence stretches, ARE, INS, CRS sequence elements, cryptic splice donor and acceptor sites, and branch points, are eliminated or reduced in number from the antigen-encoding sequence. In one embodiment, a suitable human codon-optimized gD sequence comprises the sequence of SEQ ID NO: 1.

The following are illustrative examples of additional antigens that may be used to induce protective immunity against other pathogens.

The antigens may be derived from HIV-1, (such as tat, nef, gp120 or gp160, gp40, p24, gag, env, vif, vpr, vpu, rev), human herpes simplex virus type 1 (HSV-1)(such as gH, gL gM gB gC gK gE or gD or derivatives thereof or Immediate Early protein such as ICP27, ICP 47, ICP 4, ICP36), cytomegalovirus, especially Human, (such as gB or derivatives thereof), Epstein Barr virus (such as gp350 or derivatives thereof), Varicella Zoster Virus (such as gpI, II, III and IE63), or from a hepatitis virus such as hepatitis B virus (for example Hepatitis B Surface antigen or Hepatitis core antigen or pol), hepatitis C virus antigen and hepatitis E virus antigen, or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18; *e.g*. L1, L2, E1, E2, E3, E4, E5, E6, E7 proteins), flaviviruses (*e.g*. Yellow Fever Virus, Dengue Virus, Tick-borne encephalitis virus, Japanese Encephalitis Virus) or Influenza virus cells such as HA, NP, NA, or M proteins, or combinations thereof), or antigens derived from bacterial pathogens such as *Neisseria spp,* including *N. gonorrhea* and *N. meningitidis* (*e.g.,* transferrin-binding proteins, lactoferrin binding proteins, PilC, adhesins); *S. pyogenes* (for example M proteins or fragments thereof, C5A protease), *S. agalactiae, S. mutans; H. ducreyi; Moraxella spp,* including *M. catarrhalis,* also known as *Branhamella catarrhalis* (for example high and low molecular weight adhesins and invasins); *Bordetella spp,* including *B. pertussis* (for example pertactin, pertussis toxin or derivatives thereof, filamenteous hemagglutinin, adenylate cyclase, fimbriae), *B*. *parapertussis* and *B. bronchiseptica; Mycobacterium spp.,* including *M. tuberculosis* (for example ESAT6, Antigen 85A, -B or -C, MPT 44, MPT59, MPT45, HSP10, HSP65, HSP70, HSP 75, HSP90, PPD 19 kDa [Rv3763], PPD 38 kDa [Rv0934]), *M. bovis, M. leprae, M. avium, M. paratuberculosis, M. smegmatis; Legionella spp,* including *L. pneumophila; Escherichia spp,* including enterotoxic *E. coli* (for example colonization factors, heat-labile toxin or derivatives thereof, heat-stable toxin or derivatives thereof), enterohemorragic *E. coli,* enteropathogenic *E*. *coli* (for example shiga toxin-like toxin or derivatives thereof); *Vibrio spp,* including *V. cholera* (for example cholera toxin or derivatives thereof); *Shigella spp,* including *S. sonnei, S. dysenteriae, S. flexnerii; Yersinia spp,* including *Y. enterocolitica* (for example a Yop protein), *Y. pestis, Y. pseudotuberculosis; Campylobacter spp,* including *C. jejuni* (for example toxins, adhesins and invasins) and *C*. *coli; Salmonella spp,* including *S*. *typhi, S. paratyphi, S. choleraesuis, S. enteritidis; Listeria spp.,* including *L. monocytogenes; Helicobacter spp,* including *H. pylori* (for example urease, catalase, vacuolating toxin); *Pseudomonas spp,* including *P. aeruginosa; Staphylococcus spp.,* including *S. aureus, S. epidermidis; Enterococcus spp.,* including *E. faecalis, E. faecium; Clostridium spp.,* including *C*. *tetani* (for example tetanus toxin and derivative thereof), *C*. *botulinum* (for example botulinum toxin and derivative thereof), C. *difficile* (for example clostridium toxins A or B and derivatives thereof); *Bacillus spp.,* including *B. anthracis; Corynebacterium spp.,* including *C*. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E. equi* and the agent of the Human Granulocytic Ehrlichiosis; *Ricketsia spp,* including *R. rickettsii; Chlamydia spp.,* including *C*. *trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii* (for example SAG2, SAG3, Tg34); *Entamoeba spp.,* including *E. histolytica; Babesia spp.,* including *B. microti; Trypanosoma spp.,* including *T. cruzi; Giardia spp.,* including *G. lamblia; Leshmania spp.,* including *L. major; Pneumocystis spp.,* including *P*. *carinii*; *Trichomonas spp.,* including *T. vaginalis; Schisostoma spp.,* including *S*. *mansoni,* or derived from yeast such as *Candida spp.,* including *C*. *albicans; Cryptococcus spp.,* including *C*. *neoformans.*

In some cases, two or more antigens may be used in the immunization methods and kits herein. In the priming preparations, the two or more antigens may be fusion proteins, in which either the full-length antigenic proteins or immunogenic fragments thereof are expressed from a single open-reading frame (*e.g*. expressed as a single transcript). In other cases, the two or more antigens may be expressed from different open-reading frames (*e.g*. expressed as separate transcripts) under the control of a single promoter or different promoters. In the boosting preparations, the two or more antigens may be present as a mixture of antigens or as one or more fusion proteins. The two or more antigens may be from a single pathogen or multiple pathogens.

Other preferred specific antigens for *M. tuberculosis* are for example Rv2557, Rv2558, RPFs: Rv0837c, Rv1884c, Rv2389c, Rv2450, Rv1009, aceA (Rv0467), PstS1, (Rv0932), SodA (Rv3846), Rv2031c 16 kDal., Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSI, DPV-MTI-MSL-mTCC2, TbH9-DPV-MTI (WO 99/51748). Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S. pneumoniae* (PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp.,* including *H. influenzae type B* (for example PRP and conjugates thereof), non typeable *H. influenzae,* for example OMP26, high molecular weight adhesins, P5, P6, protein D and lipoprotein D, and fimbrin and fimbrin derived peptides (U.S. Pat. No. 5,843,464) or multiple copy variants or fusion proteins thereof.

The antigens that may be used may further comprise antigens derived from parasites that cause Malaria. For example, preferred antigens from *Plasmodia falciparum* include RTS,S and TRAP.RTS is a hybrid protein comprising substantially all the C-terminal portion of the circumsporozoite (CS) protein of *P. falciparum* linked via four amino acids of the preS2 portion of Hepatitis B surface antigen to the surface (S) antigen of hepatitis B virus. Its full structure is disclosed in the International Patent Application No. PCT/EP92/02591, published under Number WO 93/10152 claiming priority from UK patent application No. 9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred case is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in *Plasmodium spp.*

Vaccines herein may also be used for the prophylaxis or therapy of chronic disorders in addition to allergy or infectious diseases. Such chronic disorders are diseases such as asthma, atherosclerosis, and Alzheimers and other autoimmune disorders. Vaccines for use as a contraceptive are also contemplated.

Antigens relevant for the prophylaxis and the therapy of patients susceptible to or suffering from Alzheimer neurodegenerative disease are, in particular, the N terminal 39-43 amino acid fragment (Aβ, the amyloid precursor protein and smaller fragments. This antigen is disclosed in the International Patent Application No. WO 99/27944--(Athena Neurosciences).

Potential self-antigens that could be included as vaccines for auto-immune disorders or as a contraceptive vaccine include: cytokines, hormones, growth factors or extracellular proteins, more preferably a 4-helical cytokine, most preferably IL 13. Cytokines include, for example, IL1, IL2, IL3, IL4, IL5, IL6, IL7, IL8, IL9, IL10, IL11, IL12, IL13, IL14, IL15, IL16, IL17, IL18, IL20, IL21, TNF, TGF, GMCSF, MCSF and OSM. 4-helical cytokines include IL2, IL3, IL4, IL5, IL13, GMCSF and MCSF. Hormones include, for example, luteinising hormone (LH), follicle stimulating hormone (FSH), chorionic gonadotropin (CG), VGF, GHrelin, agouti, agouti related protein and neuropeptide Y. Growth factors include, for example, VEGF.

The nucleic acids used in the priming preparation may be RNA or DNA including genomic DNA, synthetic DNA or cDNA. Preferably the nucleotide sequence is a DNA sequence and most preferably, a cDNA sequence. In order to obtain expression of the antigenic peptide within mammalian cells, it is necessary for the nucleotide sequence encoding the antigenic peptide to be presented in an appropriate vector system. By "appropriate vector" as used herein is meant any vector that will enable the antigenic peptide to be expressed within a mammal in sufficient quantities to evoke an immune response.

For example, the vector selected may be a plasmid, a phagemid or a viral vector. The vector may comprise promoter and polyadenylation/transcriptional termination sequences arranged in the correct order to obtain expression of the antigenic peptides. In on embodiment, the vector is a plasmid (*e.g*. pDNAVACC). The construction of vectors which include these components and optionally other components such as enhancers, restriction enzyme sites and selection genes, such as antibiotic resistance genes, is well known to persons skilled in the art and is explained in detail in Maniatis et al "Molecular Cloning: A Laboratory Manual", Cold Spring Harbour Laboratory, Cold Spring Harbour Press, Vols 1-3, 2nd Edition, 1989.

The nucleic acid in the priming preparation is a vector encoding an HSV-2 antigen under the control of a promoter. As used herein, "under the control of" or "operably linked" means that the promoter is in the correct location and orientation in relation to a polynucleotide encoding the antigen to control the initiation of transcription by RNA polymerase and expression of the polynucleotide. Suitable promoters for use in the vector include, but are not limited to, human cytomegalovirus (CMV) immediate early gene promoter, the SV40 early promoter, the Rous sarcoma virus long terminal repeat, RNA pol I, pol II, and pol III promoters. In a preferred embodiment, the promoter is a CMV immediate early gene promoter. The sequence encoding the HSV-2 antigen is codon-optimized for expression in mammalian cells, such as human cells. In certain embodiments, the vector encodes a full-length, human codon-optimized gD glycoprotein sequence comprising a sequence of SEQ ID NO: 1.

The inventors have shown that high expression of HSV-2 gD can be achieved through the use of optimized humanized codons and the use of a strong promoter, which results in an increase in immunogenicity while lowering the frequency and dose of DNA required for priming (see Examples). Previous work demonstrated that 3 doses of 100-200 µg of gD DNA were required to provide protection from live HSV-2 challenge in contrast to the 0.5-5 µg of DNA used the studies described in Examples 1-3 (29,41).

A vector carrying nucleic acids encoding an antigenic peptide can be administered in a variety of manners. It is possible for the vector to be administered in a naked form (that is as naked nucleotide sequence not in association with liposomal formulations, with viral vectors or transfection facilitating proteins) suspended in an appropriate medium, for example a buffered saline solution such as PBS and then injected intramuscularly, subcutaneously, intradermally or mucosally or administered using gene gun or other electronic (*i.e*., electroportation) devices. It is additionally possible for the vectors to be encapsulated by, for example, liposomes or within polylactide co-glycolide (PLG) particles for administration via the nasal or pulmonary routes. In preferred embodiments, the vector carrying nucleic acids encoding an HSV-2 antigen is administered in a naked form via an intramuscular route.

It is also possible for intradermal administration of the vector, preferably via use of gene-gun (particularly particle bombardment) administration techniques. Such techniques may involve coating of the vector on to gold beads which are then administered under high pressure into the epidermis, such as, for example, as described in Haynes et al. J. Biotechnology 44: 37-42 (1996).

Recombinant viral vectors can also be used to deliver DNA antigens. Advantages to this approach include abundant expression of DNA-encoded proteins in multiple cell types, stronge enhancement of CTL responses and the ability of the virus to encode multiple genes. Vaccinia virus (including modified virus Ankara) and adenovirus (non-replicating) are two popular viruses used for vaccine development (Im and Hanke, Expert, Rev. Vaccines 3:S89-S97 (2004); Basak et al., Viral Immunol. 17:182-196 (2004)).

Recent modifications of DNA vaccines include the development of minigenes encoding single CTL epitopes, the use of gene-encoded targeting signals to allow more efficient presentation of epitopes by the MHC-pathway and the generation of secreted proteins to target MHC class II pathway (Doria-Rose and Haigwood, Methods 31:207-216 (2003); Leifert et al., Immunol. Rev. 199:40-53 (2004)).

The boosting preparation comprises the antigen encapsulated in liposomes. In certain embodiments, the antigen encapsulated in liposomes in the boost preparation is the extracellular domain of gD comprising amino acids 1-314 of gD protein. In other embodiments, the antigen is the extracellular domain of gD comprising the amino acid sequence of SEQ ID NO: 2.

Liposomes have several potential advantages as delivery platforms for vaccines. Encapsulation of antigens within liposomes sequesters these antigens, thus the liposomes serve as an antigen depot capable of sustained antigen release. In addition, liposomes are biocompatible and biodegradable, and low in toxicity and immunogenicity. When appropriately sized (*e.g.,* > 0.2 µm up to 5 µm), liposomes are selectively taken up by antigen-presenting cells in the body, and have the potential to induce both humoral antibody and CTL responses. Liposomes serve as antigen carrier/vehicle as well as being an adjuvant that can be administered repeatedly without toxicity or immunogenicity.

Liposomes are structures consisting of a membrane bilayer composed of phospholipids of biological or synthetic origin, usually spherical in shape. Liposomes form naturally when phospholipids or lipids contact water. The structure of liposomes can be manipulated by methods to form them in the laboratory, including the input of energy in the form of heat, sonic energy, freeze-thaw cycles, or shear forces. The phospholipid bilayer membrane of liposomes separates and protects entrapped materials in the inner aqueous core from the outside. Both water-soluble and -insoluble substances can be entrapped in different compartments, the aqueous core and bilayer membrane, respectively, of the same liposome. Chemical and physical interaction of these substances can be eliminated because the substances are in these different compartments.

Liposomes used with the methods of the present invention can be prepared using any methods known in the art. These liposomes may have an average diameter of about 0.5 to 5 microns, about 2 to 4 microns, or about 1 to 4 microns. In some instances, liposomes of about 0.2 to 8 microns may also be useful. In certain embodiments, liposomes that may be used in the methods of the invention are anionic liposomes (e.g. negatively-charged). The charge of the liposomes can be manipulated by incorporating ionic lipids. See, e.g., U.S. Patent No. 5,290,563. For instance, incorporation of dicetyl phosphate confers a negative charge to liposomes. In one embodiment, the liposomes used in the boost preparations are anionic liposomes comprising phosphatidylcholine, cholesterol, and dicetyl phosphate. In another embodiment, the liposomes comprise phosphatidylcholine, cholesterol, and dicetyl phosphate in a ratio of 7:3:0.5 mole %. In some embodiments, the liposomes used in the boost preparations consist of lipids, *i.e.* the liposomes do not contain additional proteins, ligands, or adjuvants. For instance, in one embodiment, the liposomes are non-fusogenic liposomes (*i.e.* do not contain any proteins, such as viral proteins incorporated into the liposomal membrane).

By way of example, one method for making the liposomes that can be used in the boosting preparations in the methods of the invention is described as follows. Liposomes of the subject invention are prepared at the following lipid concentrations: Phosphatidylcholine/cholesterol/dicetyl phosphate 7/3/0.5 mole %. Dicetyl phosphate is dissolved in chloroform plus 5% of ethanol, sonicated and phosphatidylcholine and cholesterol are then added. Lipids are dried in a Labconco rotary evaporator for one hour and traces of chloroform are removed by freeze-drying with a Freezone 4.5 Freeze Dry System overnight. The lipid film is hydrated with antigen, such as gD glycoprotein from HSV-2 (gD), at a concentration of 125-300 µg/ml in 10 mM HEPES-buffer, 150 mM NaCl, pH 7.4(HBS), and filtered with a 0.2 µm nylon filter. The mixture is vortexed thoroughly and allowed to sit for 1 hour and then vortexed again to ensure the formation of multilamellar vesicles. The resultant liposomes are then subjected to three cycles of freeze-and-thaw (1 cycle-freezing for one hour and thawing for one hour at room temperature). The size of the liposomes is measured with a N4 MD Submicron Particle Size Analyzer (Coulter Electronics). The zeta-potential was measured using Zeta-Puls zeta-potential analyzer (Brookhaven Instruments) in 5 mM HEPES buffer, 1.0 mM NaCl, pH 7.4. To obtain liposomes with specific average diameters less than 2 microns, after the third freeze-thaw cycle, liposomes can be warmed in a water bath to 50°C and extruded through a polycarbonate filter with a pore size of 1.0 µm to obtain liposomes with average diameters of about 1.0 µm. The lipsomes can be further extruded to obtain small sized liposomes, for example, by further extruding the liposomes through a polycarbonate filter with pore size of 0.4 µm and finally 0.2 µm using a hand-held Avanti micro-extruder to obtain liposomes with average diameters of about 0.2 µm.

In certain embodiments, liposomes that may be used in the methods of the invention are "long circulating liposomes" (a.k.a. "sterically stabilized liposomes"), which are liposomes that comprise one or more specialized lipids that, when incorporated into liposomes, result in enhanced circulation lifetimes relative to liposomes lacking such specialized lipids. Examples of long circulating liposomes known in the art include those in which the liposome (A) comprises one or more glycolipids such as monosialoganglioside GM1 or (B) comprises one or more lipids derivatized with one or more hydrophilic polymers, such as a polyethylene glycol (PEG) moiety. While not wishing to be bound by any theory, at least for long circulating liposomes containing gangliosides, sphingomyelin, or PEG-derivatized lipids, the enhanced circulation half-life of these liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos et al. (Ann. N.Y. Acad. Sci., 1987, 507, 64) reported the ability of monosialoganglioside GM1, galactocerebroside sulfate and phosphatidylinositol to improve blood half-lives of liposomes. These findings were expounded upon by Gabizon et al. (Proc. Natl. Acad. Sci. USA, 1988, 85, 6949). U.S. Pat. No. 4,837,028 and published PCT application WO 88/04924, both to Allen et al.*,* disclose liposomes comprising (1) sphingomyelin and (2) the ganglioside GM1 or a galactocerebroside sulfate ester. U.S. Pat. No. 5,543,152 (to Webb et al.*)* discloses liposomes comprising sphingomyelin. Liposomes comprising 1,2-sn-dimyristoylphosphatidylcholine are disclosed in published PCT application WO 97/13499 (to Lim et al.).

Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, 2C1215G, that contain a PEG moiety. Illum et al. (FEBS Letters, 1984, 167, 79) noted that hydrophilic coating of polystyrene particles with polymeric glycols results in significantly enhanced blood half-lives. Synthetic phospholipids modified by the attachment of carboxylic groups of polyalkylene glycols (*e.g*., PEG) and liposomes comprising such phospholipids are described by Sears (U.S. Pat. Nos. 4,426,330 and 4,534,899). Klibanov et al. (FEBS Letts., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, *e.g*., DSPE-PEG, formed from the chemical attachment of PEG to DSPE (distearoylphosphatidylethanolamine).

Liposomes having covalently bound PEG moieties on their external surface are described in European Patent No. 0 445 131 B1 and published PCT application WO 90/04384 to Fisher. Liposome compositions containing 1-20 mole percent (mol %) of PE derivatized with PEG, and methods of use thereof, are described by Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in published PCT application WO 91/05545 and U.S. Pat. No. 5,225,212 (both to Martin et al.*)* and in published PCT application WO 94/20073 (Zalipsky et al.*)* Liposomes comprising PEG-modified ceramide lipids are described in published PCT application WO 96/10391 (Choi et al.). U.S. Pat. Nos. 5,540,935 (Miyazaki et al.) and 5,556,948 (Tagawa et al.) describe PEG-containing liposomes that can be further derivatized on their surfaces with functional moieties.

Various liposomes comprising dimyristoylphosphatidylglycerol (DMPG) have been described. Generally, however, such liposomes comprise DMPG in a mol % of about 10% or higher (see, for example, Akhtar et al. (Nucl. Acids Res., 1991, 19, 5551; Yachi et al. (Biopharm. Drug Dispos., 1996, 17, 699; and Farmer et al. (Meth. Enz., 1987, 149, 184). Liposomes having 3 mol % DMPG have been described, but such liposomes included a component (in particular, a phosphatidylcholine derivative) that is not found in the liposomal compositions of the present invention. Such phosphatidylcholine derivative components include, *e.g.,* 10 mol % distearoylphosphatidylcholine (DSPC) (Brodt et al., Cancer Immunol. Immunother., 1989, 28, 54) or 7 mol % dimyristoylphosphatidylcholine (DMPC) (Perez-Soler et al., J. Nuclear Med., 1985, 26, 743; Wasan et al., Antimicrobial Agents and Chemotherapy, 1993, 37, 246; and Li et al., Oncology Res., 1995, 7, 611).

The liposome preparation may either be freshly prepared or lyophilized for long term storage. Both preparations can be used with comparable effectiveness. The liposomes used in the methods of the invention can be all the same (*e.g*. same compositions or same size), or include more than one type of liposomes.

In certain embodiments, commercially available liposomes can be used. For example, liposomes can be made under contract by Northern Lipids Inc. (Vancouver, BC), a Contract Research Organization that specializes in the development of lipid-based liposome formulations. In certain other embodiments, liposomes of various sizes can be prepared using the methodology as described below. The resulting liposomes, depending on specific preparation protocols, are typically sized between about 0.5 µm and 5 µm, for example, at 4 µm, 1 µm, or 0.2 µm by passing preparations through a microfluidizer. Briefly, negatively-charged liposomes can be prepared at the following lipid concentrations: Phosphatidylcholine/cholesterol/dicetyl phosphate 7/3/0.5 mole %. Antigen such as HSV-2 antigen (*e.g*. gD glycoprotein of HSV-2) is incorporated into multilaminar liposomes at several concentrations for the testing of immune responses. Liposome size can be measured with a N4 MC Submicron Particle Size Analyzer (Coulter Electronics). In certain embodiments, antigen preparations can be lyophilized for storage and then reconstituted before use. Both size and ζ-potential (zeta, a measure of charge) might be measured before use. The ζ-potential was determined using Zeta-Puls ζ-potential analyzer (Brookhaven Instruments). The lipid: antigen protein ratio can be varied in some preparations in order to determine the importance of this ratio on immune responses to the specific antigen *(e.g.,* gD of HSV-2).

The priming and boosting preparations are administered in such amount as will be prophylactically or therapeutically effective. The exact quantity may vary considerably depending on the species and weight of the animal being immunized, the route of administration, the potency and dose of the priming and boosting preparations, the nature of the disease state being treated or protected against, the capacity of the subject's immune system to produce an immune response and the degree of protection or therapeutic efficacy desired. Based upon these variables, a medical or veterinary practitioner will readily be able to determine the appropriate dosage level. In the following examples, suitable doses of the DNA vector encoding gD glycoprotein from HSV-2 used in the priming preparation were determined to be about 0.5 µg to 5 µg for mice and about 100 µg for guinea pigs. These doses can be scaled to appropriate doses for use in humans by one of ordinary skill in the art without undue experimentation. For instance, suitable doses of the DNA vector encoding HSV-2 gD protein for use in humans may be from about 0.2 mg to about 100 mg, or about 0.5 mg to about 50 mg. The precise dosage will depend on the type of vector used, the promoter, the level of expression of antigen, and the type and level of codon-optimization of the antigen nucleotide sequence. In the following examples, suitable doses of liposomal-encapsulated HSV-2 gD glycoprotein used in the boosting preparation were determined to be about 15 µg/50 µl for mice and about 6 µg to 30 µg/100 µl for guinea pigs. Similar to the doses of DNA vector, these doses for the boosting preparation can be adjusted appropriately for use in humans. Suitable human doses for the liposomal-encapsulated gD antigen may be about 3 to 100 µg/100 to 400 µl, for instance about 10 µg/100 µl to about 50 µg/100 µl.

Also described is a method for providing passive, protective immunity against HSV-2 in a mammal in need thereof. In one case, the method comprises isolating serum from an immunized donor mammal and administering the isolated, immune serum to a recipient mammal, wherein the recipient mammal is thereby protected from HSV-2 infection. Preferably, the donor mammal is immunized by the methods. For instance, the donor mammal is immunized by administering to the donor mammal a priming preparation comprising a nucleic acid encoding an HSV-2 antigen and a boosting preparation comprising the antigen encapsulated in liposomes, wherein the priming preparation is administered intramuscularly and the boosting preparation is administered intranasally. In some embodiments, the donor mammal is immunized using the gD glycoprotein as the HSV-2 antigen. The isolated, immune serum can comprise HSV-2 neutralizing antibodies and/or HSV-2 antigen-specific IgG and IgA antibodies (*e.g*. anti-gD glycoprotein IgG and IgA antibodies).

In certain cases, the method further comprises isolating CD4⁺ and/or CD8⁺ T lymphocytes from the donor mammal and administering the isolated CD4⁺ and/or CD8⁺ T lymphocytes to the recipient mammal. In another case, the donor and recipient mammals are human. In still another case, the recipient mammal is at risk of HSV-2 infection.

The methods according to the present invention can be used in relation to prophylactic or treatment procedures of all mammals including, for example, domestic animals, laboratory animals, farm animals, captive wild animals and, most preferably, humans.

It is contemplated that the methods and kits herein can also be practiced with the addition of one or more adjuvants known in the art. However, in some cases, no additional adjuvant is included in the boosting preparations. The inventors have discovered that the novel methods provided herein achieve the desired immune responses without the need for any adjuvant other than liposome, thus avoiding risks and complications associated with many adjuvants, especially the bacterial toxins such as cholera toxin (CT) and the *E. coli* heat labile enterotoxin (LT).

An adjuvant is a substance or procedure which augments specific immune responses to antigens by modulating the activity of immune cells. Exemplary adjuvants include salt based adjuvants such as alum salts, bacterial-derived adjuvants like lipopolysaccharides and bacterial toxins, adjuvant emulsions that enable the slow release of antigen, agonsitic antibodies to co-stimulatory molecules, Freunds adjuvant, muramyl dipeptides, and recombinant/synthetic adjuvants. In one particular embodiment, the adjuvant is a toll-like receptor (TLR) ligand, particularly a TLR-4, such as monophosphoryl lipid A (MPL), or TLR-7 ligand, such as R837. Recently, TLR-4 and TLR-7 ligands in combination with a nanoparticle formulation have been reported to enhance and prolong antibody responses when administered with antigen following immunization (Kasturi et al. (2011) Nature, Vol. 470: 543-560). Thus, TLR-4 and/or TLR-7 ligands can be included in the priming and/or boosting preparations of the invention. Alum is the most common salt-based adjuvant used to stimulate immune responses to protein vaccines and is the only adjuvant approved for human use in the United States (Alving, Vaccine 20(3):S56-S64 (2002); Hunter, Vaccine 20(3):S7-12 (2002)). However, alum favors Th2-biased responses and does not stimulate cell-mediated immunity. Mucosal immunity can be induced through the use of bacterial toxins such as cholera toxin (CT) and the *E. coli* heat labile enterotoxin (LT), however the safety of these adjuvants is questionable (Alving, Vaccine 20(3):S56-S64 (2002); Hunter, Vaccine 20(3):S7-12 (2002)). The development of newer, safer adjuvants has recently focused on stimulating particular immune response pathways. Co-administration of cytokines, such as interferon-γ and granulocyte-macrophage colony stimulating factor (GM-CSF), has shown promise in stimulating cellular immune responses (reviewed in (Petrovsky and Aguilar, Immunol. Cell Biol. 82:488-496 (2004)). High levels of cytokines can cause toxicity however, and dosing regimens must be carefully modulated. Administration of cytokines has particular promise for DNA vaccination where genes encoding both the cytokine and antigen could be simultaneously expressed by the same vector. Additional adjuvants being explored include those that target the toll signaling pathway. CpG DNA motifs commonly found in bacterial DNA are potent activators of cellular immune responses, and newer generation DNA-based vaccines often encode multiple CpG motifs (reviewed in (Petrovsky and Aguilar, Immunol. Cell Biol. 82:488-496 (2004)).

This invention is further illustrated by the following additional examples that should not be construed as limiting. Those of skill in the art should, in light of the present disclosure, appreciate that many changes can be made to the specific embodiments which are disclosed and still obtain a like or similar result without departing from the scope of the invention.

### EXAMPLES

### Example 1. Heterologous Immunization Induces Synergistic HSV gD-Specific IgG

### Responses

We previously demonstrated that liposome charge and size are critical for induction of optimal responses to HBsAg when delivered intranasally (26). To confirm this observation for the gD antigen of Herpes Simplex Virus, type 2 (HSV-2), we immunized mice homologously (3 weeks apart) with either negatively or positively charged liposomes with average sizes of either 0.2 or 1-4 µm. Each liposome condition was tested with either 3 or 15 µg of incorporated gD. As observed for HBsAg, only negatively charged liposomes induced measurable gD-specific IgG responses (data not shown). We also observed that 15 µg of protein encapsulated in liposomes sized at 1-4 µm induced the highest responses (data not shown). From these experiments, the optimal liposome composition was chosen as 15 µg of gD encapsulated in negatively charged liposomes sized at 1-4 µm (hereinafter called gD LIP).

Prior research has demonstrated that vaccination with multiple, high doses of gD DNA alone is only sufficient to protect mice and guinea pigs from developing disease following a lethal challenge with HSV-2, while virus replication can be only partially contained (29,30). However, multiple, high doses (up to 3mg) of DNA vaccination alone is not similarly immunogenic in humans (31). A recent paper from the same group showed that this DNA vaccine raised HSV-specific T-cell responses, in what the authors termed "immuneseronegatives" (32). As these were human studies, it is not known how effective the seronegative, cellular-positive responses might be in preventing or modifying subsequent challenge.

To develop a vaccine that will be effective in the human population, we hypothesized that a prime and boost vaccination regimen that relies on synergistic responses (including mucosal immune responses) after heterologous immunization with both the DNA and the antigen-liposome components of the vaccine would have a higher chance of success. To produce the gD vaccine, the DNA sequence encoding gD was synthesized *in vitro* to generate a gene sequence fully optimized for expression in mammalian cells (GeneArt; North Carolina). The synthesized gene (SEQ ID NO: 1) was cloned into the DNA vaccine-specific vector, pDNAVACC (Nature Technology; Nebraska) under the control of a cytomegalovirus immediate early (CMV IE) promoter and sequenced to confirm identity. Efficient expression of HSV-2 gD protein by the gD DNA vaccine construct was confirmed by both Western blot and ELISA (data not shown). The gD antigen was purchased from Vybion (Ithaca, New York). The antigen was produced in *Pichia pastoris* and comprised the extracellular domain of gD (SEQ ID NO: 2). gD encapsulated liposomes were prepared as previously described (26).

To test our hypothesis, we first titrated the gD DNA vaccine in mice to determine the optimal dose of vaccination. Female Balb/c mice were immunized intramuscularly with various doses of the gD DNA vaccine (0.05-50 µg of gD DNA). Three weeks after immunization serum was collected and pooled from immunized mice and total IgG responses to gD were measured using an ELISA assay. At the second lowest vaccination dose (0.25 µg of DNA), 4/6 mice developed antibodies to gD. Higher doses of DNA resulted in a dose dependent response from all animals (Figure 1A). Mice immunized with 0.5, 5 or 50 µg of gD DNA had gD-specific antibody titers of 28, 94 and 203 µg/ml IgG, respectively. This was greater than the IgG response seen in mice that had been infected with HSV-2 viruses (1000 pfu) for 5 weeks (∼20 µg/ml total IgG; data not shown). These results indicate that the DNA vaccine expresses gD protein in sufficient quantities to elicit antigen-specific antibody responses.

To determine whether the DNA portion of the vaccine was protective in mice against disease and mortality, we challenged mice vaccinated with the gD DNA vaccine alone with either a low (5 LD50) or high (100 LD50) dose of HSV-2. As shown in Figure 1B, all but the lowest dose of DNA vaccine provided protection from challenge with 5 LD50 of HSV-2. Greater than 50% of animals immunized with all but the two highest doses of DNA vaccine died or were moribund within 2 weeks after infection with 100 LD50 of HSV-2 (Figure 1C). The higher dose of viral challenge helped reveal that protection against HSV-2 infection was dependent upon the dose of DNA vaccination: the greater the concentration of DNA vaccine, the greater the number of animals that survived. Based upon these results, we chose 0.5 µg and 5.0 µg as low (suboptimal) and high DNA vaccine doses to be used in subsequent studies.

To test the ability of the full vaccine (gD DNA+ gD LIP) to induce immune responses, female Balb/c mice were immunized heterologously by priming with an intramuscular (*i.m*.) injection of 0.5 µg of gD DNA (given on days 0 and 2) followed by an intranasal (*i.n*.) boost of gD LIP (50 µl total dose per mouse in both nostrils) given 3 weeks later. To evaluate the contribution from each of the vaccine components, separate groups of mice were immunized with either gD DNA followed by empty liposomes (gD DNA) or empty vector followed by gD LIP (gD LIP). In addition, we tested the effect of increasing the priming dose of DNA vaccine by immunizing an additional group of mice with 5 µg gD DNA+ gD LIP. Naïve mice served as a negative control. Serum and vaginal antibody responses were measured by ELISA two weeks after the liposome immunization. The results are shown in Figure 2.

Serum IgG was barely detectable after a single vaccination with either of the components alone (Figure 2A). In contrast, IgG levels after immunization with 0.5 µg gD DNA+ gD LIP reached greater than 1100 µg/ml, demonstrating a clear synergistic response. Animals immunized with 5 µg of gD DNA+ gD LIP had serum antibody responses two times that of animals immunized with 0.5 µg of gD DNA+ gD LIP (2393 µg/ml and 1151 µg/ml, respectively). The difference in IgG levels after immunizing with either component alone versus both components in combination was highly significant (p < 0.0001). Vaginal antibody responses (IgG and IgA) followed a similar pattern (Figure 2B), however, the differences did not reach statistical significance. When the gD liposome boost was delivered i.m. after DNA priming (0.5 µg dose) only a marginal increase in serum IgG was observed in the absence of any mucosal responses (data not shown).

A plaque-based Neutralization assay was used to compare the ability of serum antibodies to neutralize whole HSV-2 virus. Neutralizing HSV-2 antibody titers were measured with a complement-dependent neutralization assay. Guinea pig complement (Biomeda; Foster City, CA) was added to the medium at a final dilution of 1:125. The complement-medium mixture was used as diluent in the preparation of virus dilutions and further serum dilutions. A 0.15-ml amount of three-fold dilutions of serum was combined with an equal volume of virus dilution (calculated to produce approximately 100 plaque-forming units) and incubated for 2 hours at 37° C. After removal of growth medium, 0.3 ml of each serum-virus mixture was added to duplicate Vero cell cultures grown to near confluence in 35-mm tissue culture plates. These plates were incubated and shaken at room temperature for 1 h. The inoculum was removed and medium containing 2% FBS and 7.5 µg/ml pooled human immunoglobulin (Sigma; St Louis, MO) was added to each plate. Plates were incubated at 37° C in a CO₂ incubator for two days, the culture medium discarded and cells were fixed and stained with 0.5% methylene blue in 70% methanol. Plaques were counted and the percentage of plaque reduction was measured compared to the control (virus incubated without serum).

Neither serum collected from naïve mice, mice immunized with 0.5 µg gD DNA alone or gD LIP alone induced measureable neutralizing antibody (data not shown). In contrast, serum samples collected from mice immunized with 0.5 µg gD DNA+ gD LIP strongly neutralized HSV-2, resulting in a 70% reduction in the number of plaques seen when the serum was diluted 1:150. Neutralizing activity could be enhanced using 10× more priming dose of gD DNA (5 µg), followed by the same gD LIP boost procedure. Pooled serum samples from mice vaccinated with 5 µg gD DNA+ gD LIP showed a 94% reduction in plaque number after a 1:150 dilution of the sample.

The results of this series of experiments show that a heterologous immunization entailing intramuscular vaccination with a nucleic acid encoding the gD antigen followed by an intranasal boost of the protein form of the antigen encapsulated in liposomes produces a synergistic serum and mucosal neutralizing antibody response to the antigen.

### Example 2. Heterologous Immunization Induces Th1 to Balanced Th1/Th2 Helper Responses

The ratio of antigen-specific IgG1 and IgG2a antibodies is used to characterize the T helper type bias of an immune response (26,33,34). IgG1:IgG2a ratios <0.5 indicate a Th1-biased immune response, while a ratio of ≥2.0 indicates a Th2-biased immune response. Ratios between 0.5 and 2.0 indicate a mixed response.

To evaluate the contribution from each vaccine component (*e.g*. gD DNA and gD LIP) and characterize the type of immune response induced by the heterologous immunization protocol, we used quantitative ELISA assays to determine the IgG1:IgG2a ratio obtained after administration of each individual component and the two components combined. We also compared results after immunization with either 0.5 µg gD DNA or 5µg gD DNA (Figure 3). Immunization with 0.5 µg gD DNA or gD LIP alone resulted in IgG1:IgG2a ratios of 0.84 and 27.9, respectively, indicating mixed and Th2-biased responses. The ratio after immunization with 5 µg of gD DNA alone was 0.39 indicating a Th1-biased response. The ratios after immunization with both components were 0.47 and 0.33, respectively, after priming with 0.5 µg or 5 µg of gD DNA.

These results demonstrate that: (1) gD DNA alone immunization induces a mixed or Th1- biased response depending upon the amount of DNA used; (2) gD LIP alone induces a Th2-biased response; (3) Th1-biased responses established by DNA priming are maintained after boosting with the liposome encapsulated protein component of the vaccine.

In addition to serum and vaginal IgG, and CD4 T cells, IFN-γ is also a major protective mechanism in HSV-2 infections (35).While IFN-γ is not essential for virus clearance, it plays an important role in enhancing T cell-mediated clearance mechanisms (36). IFN-γ produced locally in the genital tract enhances virus clearance and may ultimately be important for reducing the amount of virus available to infect sensory ganglia.

Examination of specific T cell responses to gD is limited in Balb/c mice as gD-specific epitopes have yet to be defined in this strain. To determine whether whole gD protein could be used to stimulate gD specific T cells, we vaccinated mice with either 0.5 µg gD DNA, gD LIP or 0.5 µg gD DNA+ gD LIP and then infected the animals with HSV-2 two weeks after the liposome immunization. We reasoned that infection would act as an additional boost thereby enhancing immune responses and increasing the chance of detecting gD-specific T cell responses. Ten days after infection, splenocytes were isolated and incubated with 10 µg/ml of gD protein. The supernatants were collected 48 h post stimulation and examined in an ELISA assay to detect IFN-γ. As shown in Figure 4A, IFN-γ was readily detected from infected animals vaccinated with either of the components alone or by heterologous immunization, while IFN-γ release was minimal in the one surviving naïve animal (152 pg/ml). Vaccination with gD DNA alone induced the highest IFN-γ response that was significantly greater than the level observed after vaccination with gD LIP or gD DNA+ gD LIP (p < 0.05). The less IFN-γ production found in gD DNA+ gD LIP vaccinated animals may be due to the protective immune response established by the vaccination, which led to decreased virus infection and therefore limited its boosting effect on IFN-γ production. More importantly, IFN-γ secretion was enhanced in vaccinated groups of mice suggesting a gD specific T cell response was established by the immunization and vigorously recalled upon virus challenge.

In a separate experiment, we directly measured IFN-γ levels in the vaginal wash samples of naive or vaccinated mice infected with HSV-2 for varying amounts of time. Groups of mice were vaccinated with 0.5 µg DNA, 5 µg DNA, gD LIP, 0.5 µg gD DNA+ gD LIP or 5 µg gD DNA+ gD LIP and infected 2 weeks after the last immunization. Vaginal washes were collected on days 0 (before infection), 1, 3, 5 and 7 post infection and used in an ELISA assay to directly measure IFN-γ levels. IFN-γ was undetectable on days 0, 1 but increased on day 3 and peaked on day 5 post infection (Figures 4B and C), a point in time when memory T cells are responding to challenge virus and naïve immune cells undergo an innate immune response (34,35). On day 3 post infection, IFN-γ levels were lowest in naive animals. Higher levels of IFN-γ were seen in animals vaccinated with either component alone, with the highest responses seen in animals immunized with gD DNA as responses were recalled in previously primed cells. Interestingly, on day 5 IFN-γ levels in mice vaccinated with 0.5 µg DNA+ gD LIP were lower than in animals vaccinated with gD LIP alone, and levels in animals vaccinated with 5 µg DNA+ gD DNA were almost undetectable. On day 5 post infection, naive animals showed a robust innate response to virus, which were higher than that of animals vaccinated with gD DNA alone, gD LIP alone or 0.5 µg gD DNA+ gD LIP while levels in those animals vaccinated with 5 µg DNA+ gD LIP remained almost undetectable. On day 7 post infection, IFN-γ level in naïve animals became lower (64 pg/ml) than that of animals vaccinated with 0.5 µg gD DNAalone (255 pg/ml), gD LIP alone (128 pg/ml) or 0.5 µg gD DNA+ gD LIP (108 pg/ml) while the IFN-γ levels in those animals vaccinated with 5 µg DNA+ gD LIP remained undetectable. The lack of IFN- γ response in animals vaccinated with 5 µg DNA+ gD LIP may suggest that viral replication was effectively inhibited probably by neutralizing antibodies upon virus challenge, based on the correlation between virus replication and IFN-γ secretion.

### Example 3. Heterologous Immunization Protects Animals from a Lethal Dose of HSV-2 and Induces Long-Lasting Immunity

To test the ability of the heterologous immunization to provide protection from live virus, we infected mice immunized with 0.5 µg gD DNA or gD LIP alone and mice immunized with 0.5 µg gD DNA+ gD LIP. Two weeks after the last immunization, naïve and immunized animals were inoculated intravaginally with 100× LD50 of HSV-2. The clinical isolate, HSV-2 strain MS was purchased from the ATCC and grown and titered in Vero cells. Five days prior to infection, mice were injected subcutaneously with 2 mg of medroxyprogesterone (Depo-Provera, Pfizer, St. Louis, MI). On the day of infection, animals were anesthetized intraperitoneally with a ketamine/xylazine mixture and instilled intravaginally with a 20 µl suspension containing the indicated virus dose. Animals were monitored for body weight and clinical signs of disease for at least 21 days after infection. Lesions were scored according to the following scale: 0 = no visible redness or lesions, 1 = redness or mild swelling, 2 = erosions, vesicles, or moderate swelling, 3 = several large vesicles, 4 = large ulcers with severe maceration and/or urinary retention and/or hind limb paralysis. Animals that reached a clinical score of 4 were immediately euthanized. A summary of the clinical scores is shown in Table 2 and survival curves are shown in Figure 5.

**Table 2. Clinical scores are decreased in mice immunized by heterologous immunization.**

| Immunization | # of animals with clinical score (%) | | | |
|---|---|---|---|---|
| | 0 | 1-2 | >2 | Death |
| Naïve | 0 | 0 | 5 (100%) | 4 (80%) |
| 0.5 µg gD DNA | 0 | 1 (10%) | 9 (90%) | 5 (50%) |
| gD LIP | 0 | 0 | 10 (100%) | 6 (60%) |
| 0.5 µg gD DNA + gD LIP | 3 (30%) | 4 (40%) | 3 (30%) | 2 (20%) |

| | | | | |
|---|---|---|---|---|
| 0- normal; 1- redness or mild swelling; 2- raw patch, blister, moderate swelling; 3- multiple blisters or raw patches; 4- large blisters with severe and/or deep sores; and/or urinary retention; and/or hind limb paralysis. Animals demonstrating a clinical score of 4 were considered moribund and were sacrificed. n = 5 for naive group and n = 10 for all other groups. | | | | |

All infected naive animals progressed to severe signs of disease; 4/5 of the animals were moribund or died as a result of the infection. The fifth animal developed a score of 3.5. The average maximum clinical scores among infected animals immunized with 0.5 µg gD DNA or gD LIP alone were 2.7 and 3.0, respectively. No significant differences in survival were observed after vaccination with either 0.5 µg gD DNA or gD LIP alone compared to unvaccinated animals (p = 0.09 and 0.55, respectively). In contrast, mortality was significantly reduced in animals vaccinated with the heterologous regimen as compared to unvaccinated animals (p = 0.01). Only 2/10 animals (20%) that underwent heterologous immunization were moribund/died. A majority of the animals immunized with 0.5 µg gD DNA+ gD LIP developed only minor signs of disease. Three infected animals did not develop any signs of clinical disease, while four did not progress beyond a clinical score of 2.0.

In a separate experiment, animals were vaccinated with 5 µg gD DNA alone or 5 µg gD DNA+ gD LIP prior to infection. In this experiment, animals were only held until 10 days post infection so that spleens could be harvested. At the time of sacrifice of the animals (day 10), 5/6 animals vaccinated with 5 µg gD DNA alone had a clinical score of 3 while the sixth animal had a clinical score of 2. Importantly, only 1/5 animals immunized with 5 µg gD DNA+gD LIP showed clinical score of 4, while the remaining four animals showed no clinical signs of disease.

Vaginal samples were collected from unvaccinated naïve animals, animals immunized with 0.5 µg gD DNA or gD LIP alone, or animals immunized with both 0.5 µg gD DNA+ gD LIP on days 1, 3, 5 and 7 post infection. In addition, samples were collected from the animals immunized with 5 µg gD DNA alone or those animals immunized with 5µg gD DNA+ gD LIP that showed no clinical signs of disease by day 10 post infection. Samples were titrated in Vero cells for detection of virus. In unvaccinated animals, virus could be detected in the vagina 1 day post infection (Figure 6). Viral titers peaked at 3 days post infection and declined thereafter, paralleling IFN-γ levels (Figure 4). On days 1, 3 and 5 post infection viral replication was detected in all samples isolated from animals immunized with any of the vaccine components alone or 0.5 µg gD DNA+gD LIP. On these days viral titers were decreased to varying degrees depending upon the vaccine used, however no statistical differences were observed between these groups. In contrast, no virus could be recovered from animals immunized with 5 µg gD DNA+gD LIP that showed no clinical signs of disease (4/5 mice) at any point post infection.

To test the ability of heterologous immunization to induce long-lasting protective immunity, animals immunized with 0.5 µg dose gD DNA+ gD LIP were infected at 10 or 20 weeks after the booster immunization. Results were compared to naïve animals, and animals immunized with gD DNA alone. Figure 7 shows the results from the 10 and 20 week infections compared to those obtained in Figure 5, in which animals were infected 2 weeks after boosting. Only one naive animal (1/15; 7%) survived infection among the 3 time points that were investigated. Two weeks after the booster, 50% of the animals immunized with gD DNA alone survived infection. Immunity induced by gD DNA alone appeared to decrease over time as only 20% of animals survived infection at 10 and 20 weeks after boosting. The mortality rate at 20 weeks after vaccination was significantly different from the mortality rate seen at 2 weeks after vaccination (p = 0.048). In contrast, heterologous immunization with gD DNA+ gD LIP induced long-lasting protective immunity. At 10 and 20 weeks after boosting, 89% and 60% of animals survived infection, respectively. Comparison of mortality rates after infection at 2, 10 and 20 weeks post vaccination revealed no significant differences between all groups (p > 0.373).

Taken together, the results show that a HSV-2 vaccine comprised of a DNA prime given intramuscularly and a liposome-encapsulated boost delivered intranasally induces clear synergistic immune responses, including humoral, T cell and mucosal immunity. The vaccine is flexible and potency can be enhanced by increasing the dose of DNA vaccine. In fact, sterilizing immunity was observed in 80% of mice receiving the higher dose of DNA vaccine in combination with the liposome component. Protection is durable as >50% of animals are still protected from lethal disease 20 weeks after final immunization (primed with suboptimal DNA dose). Interestingly, this heterologous immunization protocol induces protective immune responses in the vaginal cavity, a remote mucosal site from the location of immunization.

In sum, this heterologous immunization regimen stimulated high titers of serum neutralizing antibodies, a DNA priming dose dependent T helper type response, enhanced mucosal immune responses and potent protective immunity at the portal of entry for the virus: the vaginal cavity.

### Example 4. Passive transfer of serum and CD4⁺ T cells from immunized mice protected naïve mice from HSV-2 challenge

Mice were immunized with the heterologous immunization protocol described in Examples 1-3. Specifically, mice received an intramuscular priming dose of a DNA vector encoding gD antigen followed by an intranasal boost dose of gD antigen encapsulated in liposomes. Spleens were collected from either these vaccine-immunized mice or naïve donor mice. CD4⁺ T cells were purified by FACS sorting. Due to the limited numbers of donor T cells, the recipient, naïve mice received 5 X 10⁵ CD4⁺ T cells from immunized or naive donors. Twenty four hours following the T cell transfer, the recipient mice were passively transferred with either immune serum from the vaccinated mice or naïve serum, and then challenged with 2 X 10⁴ PFU of HSV-2. The approximate LD₅₀ of this HSV-2 virus stock in progesterone-treated Balb/c mice was 1.3 X 10³ PFU.

Mice that received immune serum transfer were significantly protected from disease and death post HSV-2 challenge compared to the recipients that received naive serum (Figures 8B and C). These results suggest the immune serum is the major contributor to the observed protection. Due to the low number of T cells used, the mice that received immune CD4⁺ T cells together with naïve serum didn't show any improvement as compared to mice that received naive serum alone or naïve serum together with naïve CD4+ T cells. Interestingly, co-transfer of immune CD4⁺ T cells and immune serum provided better protection than the transfer of either immune CD4⁺ T cells or immune serum alone. Although the differences were not statistically significant, a difference was seen in body weight curves, clinical score curves, as well as viral load results (Figures 8A, B, and D). Thus, these data suggest that the immune serum alone provides a protective role, and a combination of immune serum and immune CD4⁺ T cells produces a more efficient protection against HSV-2 infection in a synergistic fashion.

### Example 5. Comparison of intramuscular and intranasal boosting strategies post gD DNA priming

To determine whether the route of administration of the boost dose affected the quality of the protective immune response induced, Balb/c mice were primed with gD DNA vaccine (*i.m*.) first and then boosted with gD Lip (gD antigen encapsulated in liposomes) either *i.n.* or *i.m.* three weeks later (Figure 9A). Two weeks post the gD Lip boost (one day prior to HSV-2 challenge), the serum and vaginal wash samples were collected from mice to detect anti-gD-specific IgA titers by ELISA. Mice that received the liposome boost *i.m.* did not show any IgA response, while mice that received gD. Lip boost *i.n.* had an average of 7.8 ± 1.3 µg/mL gD-specific IgA in the serum and 0.23 ± 0.13 µg/mL in the vaginal wash samples (data not shown). After HSV-2 challenge at two different doses (2 X 10⁴ PFU and 8 X 10⁴ PFU), the body weight (Figure 9B) and clinical score (Figure 9C) were recorded daily. Vaginal wash samples were collected and examined by plaque assay to titrate the virus titers (Figure 9D). The results show that *i.n.* delivered gD Lip boost induced more efficient protection against HSV-2 challenge two weeks post boost than the *i.m.* administered gD Lip, particularly at a higher challenge dose (Figure 9). A quicker virus clearance from the vaginal site and a more efficient protection against HSV-2 induced disease were observed in mice that received *i.n.* liposome boost compared to *i.m.* boosted mice (Figures 9C and D).

To test the hypothesis that mucosal vaccination can provide better long-lasting protection than systemic immunization, we compared the protective efficacy of mice that received liposome boost either *i.n.* or *i.m.* (17 weeks after the last immunization). Balb/c mice were primed with gD DNA vaccine (*i.m*.) first and then boosted with gD Lip (gD antigen encapsulated in liposomes) either *i.n.* or *i.m.* three weeks later (Figure 10A). Seventeen weeks post the gD Lip boost, all the animals were challenged with either 2 X 10⁴ or 8 X 10⁴ PFU of HSV-2. The body weight (Figure 10B) and clinical score (Figure 10C) were recorded daily post infection. Vaginal wash samples were collected and examined by plaque assay to titrate the virus titers (Figure 10D). The results show that mice that received the gD Lip boost intranasally were better protected than those mice that received the gD Lip boost intramuscularly.

### Example 6. A Mucosal Vaccine for Herpes Simplex Virus Type-2 induces Neutralizing Antibodies in Serum and Mucosa in Guinea Pigs

To further examine the efficacy of a heterologous immunization protocol to induce protective immunity against HSV-2, the protocol was tested in a guinea pig model. Unlike the mouse HSV-2 infection model, which lacks development of recurrences of genital herpes, the guinea pig has been used to study immune protection efficacy against both acute clinical disease and its subsequent latency and recurrence post HSV-2 infection (Stanberry (1991) Rev. Infect. Dis., Vol. 13(11); S920-S923).

First, we evaluated the capability of the vector encoding the gD antigen (*i*.*e*. gD DNA vaccine) to express the antigen in host cells. To produce the gD vaccine, the DNA sequence encoding gD was synthesized *in vitro* to generate a gene sequence fully optimized for expression in mammalian cells (SEQ ID NO: 1). The synthesized gene was cloned into the DNA vaccine-specific vector, pDNAVACC (Nature Technology; Nebraska) under the control of a cytomegalovirus immediate early (CMV IE) promoter and sequenced to confirm identity. 293T cells were transfected with empty vector or the vector containing the codon-optimized gD sequence. Expression of the gD antigen was evaluated by Western Blot analysis. As shown in Figure 11, gD DNA vaccine highly expressed the full length gD protein. The expression of gD protein from the vaccine vector was so high that a small portion of the protein antigen was detected in the supernatant of the gD DNA vaccine transfected cells. The extracellular HSV-2 gD protein comprising amino acids 1-314 of gD protein (recombinant gD protein) was produced in *Pichia pastoris,* and encapsulated in negatively-charged liposomes for the boost component of the vaccine. The extracellular HSV-2 gD protein (secreted from yeast cell culture) was confirmed to be smaller than the full length gD protein.

To test the ability of the vaccine (gD DNA prime + gD LIP boost) to induce immune responses, 10 female Dunkin Hartley guinea pigs were immunized heterologously by priming with an *i.m.* injection with 100 µg of gD DNA (given on days 0 and 2; (50 µg per day)) followed by an *i.n.* boost with 30 µg of gD LIP (100 µl total dose per guinea pig in both nostrils) given 3 weeks later (Figure 12). To evaluate the contribution from each of the vaccine components alone and together, groups of 10 guinea pigs were immunized with gD DNA followed by empty liposomes or DNA vector followed by gD LIP. Naïve guinea pigs served as a negative control.

Five weeks post immunization, the antibody responses were evaluated. Serum IgG measurements after a single vaccination with either gD DNA or gD LIP alone were 2000 µg/ml and 480 µg/ml respectively (Figure 13A). The background/basal level in naïve animals was 200 µg/ml. In contrast, serum IgG levels after immunization with gD DNA + gD LIP reached greater than 25,000 µg/mL, demonstrating a clear 10-fold synergistic response. The difference in serum IgG levels after immunizing with DNA alone versus both components in combination was highly significant (Figure 13E; p<0.0001 for serum; n=10 per group).

Vaginal IgG after a single vaccination with either gD DNA or gD LIP alone was 4.8 µg/ml and 1.3 µg/ml respectively (Figure 13B). The background/basal level in naïve animals was 1.1 µg/ml. In contrast, vaginal IgG levels after immunization with gD DNA + gD LIP reached greater than 50 µg/mL, also demonstrating a clear 10-fold synergistic response. The difference in vaginal IgG levels after immunizing with DNA alone versus both components in combination was highly significant (Figure 13F; p<0.001 for IVAG, n=10 per group). Serum and vaginal IgA antibody responses followed a similar pattern in pooled samples (Figures 13C and D) and in individuals by measurement of end point ELISA titers by geometric means: IgA mean after immunization with gD DNA alone was 310 and 60 for serum and IVAG respectively; IgA mean after immunization with both gD DNA and gD LIP was 3480 and 484 for serum and IVAG respectively (not shown).

To test whether serum from immunized guinea pigs exhibited neutralization activity against HSV-2 virus, samples were collected prior to viral challenge and analyzed in a plaque-based neutralization assay. Neutralizing HSV-2 antibody titers were measured with a complement-dependent neutralization assay. Guinea pig complement (Biomeda; Foster City, CA) was added to DMEM medium at a final dilution of 1:125. The complement-medium mixture (containing 5-10% FBS final) was used as diluent in the preparation of virus dilutions and sample dilutions. For serum samples, a 0.15-ml amount of 2-fold dilutions was combined with an equal volume of virus dilution (containing approximately 100 PFU). For IVAG samples, a 0.2-mL amount of 2-fold dilutions was combined with 20 µL of virus dilution (containing approximately 100 PFU). The mixture was incubated for 1 hour at 37° C. After removal of growth medium, 0.2 ml of each sample-virus mixture was added to duplicate Vero cell cultures grown to near confluence in 6 well plates. These plates were incubated with intermittent rocking at 37° C for 90 min. The plates then were overlaid with overlay media (1:1 mixture of 1% agarose and DMEM containing 10% heat inactivated FBS; 1X Pen/Strep; 1X L-Glutamine and 1X Fungizone). Plates were incubated in a CO₂ incubator for two days, and then stained with staining media (the overlay media containing 1.2%-1.5% of 1% neutral-red). Plaques were counted one day post staining, and the percentage of plaque reduction was measured compared to the control (virus incubated without sample).

Samples collected from naïve guinea pigs, guinea pigs immunized with gD DNA alone or gD LIP alone did not induce measureable neutralizing antibody (Figure 14A). Strikingly, samples collected from guinea pigs immunized with gD DNA + gD LIP strongly neutralized HSV-2 in a dose dependent manner, resulting in a 88%, 98% and 100% reduction in the number of plaques at 1:200, 1:100 and 1:50 dilutions respectively, compared to virus alone with no sample added. In order to examine whether the *i.m* prime and *i.n.* boost vaccine strategy could produce a mucosal neutralization response in vaginal secretions, the neutralization assay was also used to compare the ability of vaginal antibodies to neutralize whole HSV-2 virus. Again, samples collected from naïve guinea pigs, guinea pigs immunized with gD DNA alone or gD LIP alone failed to induce measureable neutralizing antibody (Figure 14B), In contrast, vaginal samples collected from guinea pigs immunized with gD DNA + gD LIP strongly neutralized HSV-2 in a dose dependent manner, resulting in a 96.5% reduction in the number of plaques seen when undiluted pooled samples were used (Figure 14B), Figure 14C shows averages +/-SEMs for vaginal samples at a 1:2 dilution. The reduction in plaque number was 50.8% (significance: p<0.001 in relation to virus alone with no sample added) when a 1:2 dilution of individuals samples were used. To our knowledge this is the first time that mucosal vaginal secretions from animals immunized with any non-replication vaccine delivery system have been shown to neutralize HSV-2 on detectable levels.

### Example 7. Heterologous Immunization Using gD Antigen Protects Guinea Pigs from a High Dose of HSV-2 Mucosal Challenge

We infected guinea pigs immunized with gD DNA alone, gD LIP alone, or gD DNA+gD LIP (see Example 6 for details of immunization) to test the ability of the heterologous immunization to provide protection from live virus. Two weeks after the last immunization, naïve and immunized animals were inoculated intravaginally with 2 X 10⁶ pfu of HSV-2. Animals were monitored for body weight and clinical signs of disease for 90 days after infection. Lesions were scored according to the following scale: 0 = no visible redness or lesions, 1 = redness or mild swelling, 2 = erosions, vesicles, or moderate swelling, 3 = several large vesicles, 4= large ulcers with severe maceration and/or urinary retention and/or hind limb paralysis. Animals that reached a clinical score of 4 were immediately euthanized. Vaginal swabs were taken every other day for 7 days post infection (p.i.). After analysis of viral load in the genital tract from swabs taken from 7 days immediately following the virus challenge it was determined that 4 guinea pigs (one from each group) had not been successfully infected and they were subsequently excluded from further analysis. Figure 15A shows the average clinical scores per group during the acute phase of disease and a summary of the clinical scores is shown in Table 3. Survival curves are shown in Figure 15B.

**Table 3: Clinical Scores Are Decreased in Guinea Pigs Immunized by Heterologous Immunization**

| **Immunization** | **Number of Animals with Clinical Disease** | | | |
|---|---|---|---|---|
| | **Score 0** | **Score 1-2** | **Score >2** | **Death** |
| Naïve | 0 | 0 | 9 | 5 |
| gD DNA | 4 | 4 | 1 | 0 |
| gD LIP | 0 | 0 | 9 | 7 |
| gD DNA+gD LIP | 8 | 1 | 0 | 0 |

| | | | | |
|---|---|---|---|---|
| Disease severity was scored using the following scale: (0) no symptoms, (1) vaginal erythema (redness or swelling), (2) single or few small vaginal vesicles, (3) several large or fused vaginal herpetic lesions, (4) severe genital ulceration and/or hind limb paralysis, and (5) found dead. Animals that reach a moribund state or exhibit a score of (4) were immediately euthanized. n=9 for each group. Guinea pigs immunized with gD DNA+gD LIP showed significantly reduced clinical score signs of disease compared to unvaccinated and gD DNA or gD LIP alone immunized animals (p=0.01, Log-rank test). | | | | |

Of the infected naive animals 9/9 progressed to severe signs of disease; 5/9 of the animals were moribund or died as a result of the infection, with an average maximum clinical score of 3.5 (Figure 15 and Table 3). The average maximum clinical scores among infected animals immunized with gD DNA or gD LIP alone were 0.45 and 3.95, respectively. Four of the nine animals immunized with gD DNA alone progressed to a clinical score of 1-2, and only one was >2. None of these animals were moribund/died. Surprisingly, 9/9 animals immunized with gD LIP alone scored greater than 2, and 7 were moribund/died, suggesting a possible enhanced immune pathology in guinea pigs associated with a Th-2 biased immune response that we observed previously in the mouse model. Excellent immune protection, based on clinical score and mortality data, was achieved in animals vaccinated with the heterologous regimen gD DNA+gD LIP, as compared to unvaccinated and gD LIP alone immunized animals (p=0.01, Log-rank test). A great majority (8/9) of the animals immunized with gD DNA+gD LIP showed no signs of disease while only one animal exhibited a marginal score 0.5-1 briefly on days 21 and 22 post infection (Figure 15A and Table 3). None of the animals that underwent heterologous immunization were moribund/died (Figure 15B).

Vaginal samples were collected from all the animals on days 1, 3, 5 and 7 post HSV-2 challenge. Samples were titrated in Vero cells for detection of virus. Figure 16 shows that virus could be detected in the vagina of all groups peaking on day 1 post infection and decreasing to varying degrees until day 7. However compared to unvaccinated animals, viral titers in animals vaccinated with both gD DNA+gD LIP on day 3 and day 7 were 2.37 and 0.94 logs lower (P=<0.01). In fact, no virus was detected on day 7 for heterologously vaccinated guinea pigs (detection level = 1.7 log PFU/ml) indicating early clearance of the virus from the genital tract. Paralleling the clinical score and survival curve data, vaginal titers of animals on days 3 and 7 vaccinated with gD DNA alone were also 1.2 logs lower than unvaccinated guinea pigs on both days (P= 0.001). No significant difference was observed between animals vaccinated with gD LIP and unvaccinated animals.

### Example 8. Heterologous Vaccination Protects Guinea Pigs from Latent Infection and Reactivation of HSV-2 during the Recurrent Phase of Disease

To test if vaccinated animals exhibited signs of recurrent disease symptoms following acute disease (approximately 15 days post-infection), guinea pigs were kept for 90 days post-infection and monitored daily. Figure 17 shows the accumulated recurrence per guinea pig for each group adjusted for days that animals exhibited recurrences. Animals that were vaccinated with gD DNA or gD LIP alone did not show a significant reduction in the number of HSV-2 reactivation events compared to unvaccinated animals. Strikingly however, animals vaccinated with gD DNA+gD LIP showed no signs of recurrent events except for one animal that scored 0.5-1 on days 21 and 22 post infection, paralleling the acute phase where no signs of disease were also noted. The results demonstrate the robust nature of the vaccine preventing initial acute infection and any subsequent reactivation or recurrence.

At the end of the study on day 90 post infection the animals were sacrificed and dorsal root ganglia (DRG) were harvested from the lumbosacral region of the spinal cord from surviving animals to test for the presence of latent HSV-2 viral genomes. Genomic DNA was extracted and viral DNA was measured by real-time quantitative PCR (qPCR) using HSV-2 glycoprotein G (gG) - specific primers. Figure 18 shows that for naïve guinea pigs, the average copy number of viral genomes per 100 ng of total DNA was 128 and animals vaccinated with gD LIP alone had an average of 154.5 copies. Interestingly, the amount of latent viral DNA in animals vaccinated with gD DNA alone was significantly lower (27.56 copies; p<0.01). Notably, only one animal vaccinated with gD DNA+gD LIP exhibited any latent DNA (7 copies/100 ng) above the detection limit of 5 copies/100 ng. Taken together with the inhibition of acute and recurrent disease afforded by the mucosal vaccine, these results suggest that HSV-2 virus is unable to spread from genital mucosa to sensory nerve nuclei in dorsal root ganglia in guinea pigs vaccinated by the heterologous immunization protocol.

To evaluate the longevity of the protective immune response induced by the heterologous immunization protocol, female Dunkin Hartley guinea pigs were immunized using the heterologous immunization protocol described above and challenged with live HSV-2 virus 15 weeks following the liposomal boost. Specifically, animals were immunized heterologously by priming with an *i.m.* injection with 100 µg of gD DNA on days 0 and 2 followed by an *i.n.* boost with either 6 or 30 µg of gD LIP (extracellular gD domain encapsulated in anionic liposomes) given 3 weeks later. Fifteen weeks following the gD LIP boost, all the animals were challenged with 2 x 10⁶ PFU of HSV-2. Successfully immunized and infected animals were clinically observed for 35 days post infection. Two different liposome batches (old and the new) of gD LIP vaccines (30 µg/100 µl), two different intranasal inoculation volumes (100 µl vs. 300 µl) and two different doses of gD Lip vaccine (6 vs. 30 µg/100 µl) in raising long-term protection were compared. The percentages of survival of each group of guinea pigs are shown in Figure 19. Animals boosted with either old gD LIP or new gD Lip were very well protected from a high-dosed HSV-2 challenge. There was no difference of survival between the groups *i.n.* boosted with gD Lip of either 30 µg in 100 µL or 6 µg in 100 µL. After 30 µg in 100 µL was diluted by 200 µL of water to be 30 µg in 300 µL and *i.n.* delivered, earlier and worsened survival were observed. The optimal mucosal HSV-2 formula for guinea pigs was determined to be *i.m.* injection with 100 µg of gD DNA on days 0 and 2 followed by an *i.n.* boost with 6 to 30 µg of gD Lip given 3 weeks later.

In summary, the results of these studies demonstrate the efficacy of the heterologous immunization protocol for inducing protective immunity against HSV-2. This heterologous immunization regimen meets many criteria for a potent HSV-2 vaccine: the vaccine stimulates high titers of serum and vaginal antibodies with high neutralizing activities; it significantly reduces the viral vaginal load and prompts viral clearance during the acute phase; it completely protects animals from developing clinical signs of disease up to 90 days post HSV-2 challenge; and more strikingly, it prevents establishment of HSV-2 latency in dorsal root ganglia. The DNA prime and liposome boost strategy is effective as well as safe to elicit mucosal immune responses and protection against HSV-2.

### Example 9. Therapeutic Efficacy of a Mucosal Herpes Simples Virus Type-2 Vaccine in Guinea Pigs

In this example, we tested whether a vaccine comprised of an intramuscularly administered DNA vaccine and an intranasally administered liposomal-encapsulated protein antigen, has therapeutic efficacy in containing genital herpes recurrence in guinea pigs previously infected with HSV-2. The guinea pig model is one of the most widely used animal models for studying genital herpes (Stanberry et al. (1985) Intervirology, Vol. 24(4): 226-231; Stanberry et al. (1982) J Infect Dis, Vol. 146(3): 397-404). Similar to humans, the guinea pig model features an initial acute infection with severe genital lesions followed by spontaneous recurrent events, and has been widely used for the evaluation of both prophylactic and therapeutic vaccines.

To establish guinea pigs infected with HSV-2 exhibiting clinical disease parameters to monitor the effect of a therapeutic vaccine, we initially inoculated animals with 4 x 10⁴ to 1 x 10⁶ pfu HSV-2 (strain MS) intravaginally. Three groups of animals (n=16/group) were infected with three different viral doses: 4 x 10⁴, 2 x 10⁵ and 1 x 10⁶ pfu. Clinical signs of disease and morbidity were monitored daily post infection. Vaginal swabs collected on day 2 and day 4 post infection were analyzed by plaque assay to confirm if viral infection and replication successfully occurred in the vaginal cavity. It was determined that 5 out of 48 guinea pigs exhibited no signs of viral infection, indicating the successful infection rate was 89.6%. Of the remaining animals, a total of 13 animals were found dead or moribund due to the virulent HSV-2 infection. The remaining 30 successfully infected guinea pigs displayed clinical signs of disease ranging from a clinical score of 2-3 during the acute phase, and were randomly divided into two groups (n=15/group) on day 28 post infection.

To test the ability of the vaccine (gD DNA vaccine prime + gD LIP boost) for therapeutic effect, the guinea pigs were immunized with the vaccines and vehicle controls as indicated in the schematic in Figure 20A. For the prime dose, gD DNA vaccine encoding a codon-optimized, full-length gD glycoprotein gene under the control of a cytomegalovirus immediate early promoter (group 1) or plasmid vector (group 2) was administered intramuscularly on Days 28 and 30 (total 100 µg DNA per guinea pig; 50 µg per day). For the boost dose, negatively-charged liposomes encapsulating the extracellular domain of the gD glycoprotein (gD-liposomes)(group 1) or empty liposomes (group 2) were administered 2 weeks after the DNA prime (100 µl total dose per guinea pig, 50 µl per nostril) as illustrated in Figure 20A.

Serum samples were collected from the vaccinated and unvaccinated groups of guinea pigs on day 21 three weeks after infection (7 days before the DNA prime) and on day 57 (15 days after the liposome boost). Serum IgG from pooled samples from the vaccine and vector groups before vaccination was 1.7 x 10⁴ and 1.5 x 10⁴ µg/ml, respectively. After vaccination, serum IgG in the vector/vehicle control group was not significantly increased (1.9 x 10⁴ µg/ml). However, serum levels had increased more than 10-fold in vaccinated animals to 2.1 x 10⁵ µg/ml (Figure 20B). When individual samples obtained at day 57 were analyzed post-vaccination (Figure 20C), the level of anti-HSV-2 gD IgG in the vaccine group was significantly higher than the control group (approximately 10-fold, P<0.001). Both serum samples and vaginal swab samples collected on day 57 were tested in a virus neutralization assay as described in Example 6. Vaccine treatment induced higher neutralization serum (Figure 21A) and vaginal (Figure 21B) antibody responses in guinea pigs exposed to HSV-2 infection. The results show that administration of the gD DNA and liposome vaccine in HSV-2-infected animals was very successful in breaking the barrier of pre-existing anti-HSV-2 immunity, including anti-gD specific antibodies.

After the primary infection, HSV-2 sets up a latent infection in the sacral nerve ganglia and reactivation of virus occurs periodically over the lifetime of an individual and may result in recurrent disease or unapparent virus shedding that occurs even in the presence of immune responses to the initial virus infection (Whitley and Roizman (2001) Lancet, Vol. 357(9267): 1513-1518). A successful therapeutic vaccine would have to prevent or markedly reduce periodic recurrences. After immunization of infected guinea pigs, the animals were observed for 60 days for evidence of spontaneous recurrent herpatic lesions. Recurrent episodes were enumerated as cumulative recurrences (appearance of lesions) per guinea pig for each group, adjusted for the number of days the recurrences were observed (Figure 22). The data indicate that the administration of the vaccine to pre-infected guinea pigs markedly reduced the number of recurrences of HSV-2 clinical disease. The Table in Figure 22 shows that post vaccination the difference between the vaccine and the control groups is highly significant (P<0.0001). The difference in recurrence rates between the two treatment groups of animals may be due to the synergistic immune responses, especially the mucosal immunity, induced by the prime and boost heterologous immunization regimen. The neutralization antibodies were enhanced in serum samples and especially in vaginal samples after immunization with the mucosal HSV-2 vaccine (Figure 21), suggesting the possible role of mucosal immunity raised by this vaccine on therapeutic efficacy.

At the end of the study, the animals were sacrificed and dorsal root ganglia (DRG) were harvested from the lumbosacral region of the spinal cord to test for the presence of latent HSV-2 viral genomes. Genomic DNA was extracted and viral DNA was measured by real-time quantitative PCR (qPCR) using HSV-2 glycoprotein G (gG)-specific primers. The guinea pig genomic DNA was measured by qPCR using guinea pig lactalbumin-specific primers. The levels of latent HSV-2 virus were expressed as copy numbers of HSV-2 per 100 ng of guinea pig genomic DNA. For the control group (DNA vector + empty liposomes), the average copy number of viral genomes per 100 ng of genomic DNA was 247.5± 98.2 (n= 14). For the vaccine group (gD DNA + gD liposomes) the average copy number of HSV-2 was 106.9 ±27.5 (n= 14). There was no statistically significant difference between the two groups (p= 0.18). It is not unexpected that the vaccine given therapeutically does not affect the latent viral copy number in the DRGs, since this is how HSV-2 evades host defenses. However, taken together with the reduced amount of recurrence observed after vaccine treatment, the results of these experiments suggest that the vaccine treatment is clinically therapeutic without eliminating viral latency.

The main obstacle for therapeutic HSV vaccines is the establishment of latency in nuclei of dorsal root ganglia which protects the virus from immune responses. Immune responses stimulated by a therapeutic vaccine likely only reach the virus after reactivation either during migration from sensory ganglia or at peripheral nerve endings and genital mucosa. As a mucosal vaccine, our heterologous gD DNA prime and gD liposome boost strategy has been shown to generate much stronger immune responses and protection than DNA vaccine alone in both mice and guinea pig models challenged by HSV-2 (see Examples 3 and 7). The synergistic immune responses, especially those at the mucosal sites, function to contain virus shedding and spreading, thereby controlling recurrence of clinical diseases related to HSV-2 reactivation.

### REFERENCES

1. Handsfield HH, Stone KM,Wasserheit JN. Prevention agenda for genital herpes. Sex Transm Dis 1999; 26(4):228-31.
2. Lafferty WE, Coombs RW, Benedetti J, Critchlow C, Corey L. Recurrences after oral and genital herpes simplex virus infection. Influence of site of infection and viral type. N Engl J Med 1987; 316(23):1444-9.
3. Mertz GJ, Benedetti J, Ashley R, Selke SA, Corey L. Risk factors for the sexual transmission of genital herpes. Ann Intern Med 1992; 116(3):197-202.
4. Wald A, Corey L, Cone R, Hobson A, Davis G, Zeh J. Frequent genital herpes simplex virus 2 shedding in immunocompetent women. Effect of acyclovir treatment. J Clin Invest 1997; 99(5):1092-7.
5. Straus S, Corey L. Herpes simplex viruses. In: Mandell GL, Douglas RG, Bennett JE, Dolin R, editors. Mandell, Douglas, and Bennett's principles and practice of infectious diseases. Philadelphia [etc.]: Churchill Livingstone; 2000. p. 1556-80.
6. Kimberlin DW. Antiviral therapy for cytomegalovirus infections in pediatric patients. Semin Pediatr Infect Dis 2002; 13(1):22-30.
7. Stamm WE, Handsfield HH, Rompalo AM, Ashley RL, Roberts PL, Corey L. The association between genital ulcer disease and acquisition of HIV infection in homosexual men. JAMA 1988; 260(10):1429-33.
8. Stanberry LR, Spruance SL, Cunningham AL, Bernstein DI, Mindel A, Sacks S, et al. Glycoprotein-d-adjuvant vaccine to prevent genital herpes. N Engl J Med 2002; 347(21):1652-61.
9. Whitley RJ, Kimberlin DW, Roizman B. Herpes simplex viruses. Clin Infect Dis 1998; 26(3):541-53, quiz 554-545.
10. Ashley RL, Dalessio J, Burchett S, Brown Z, Berry S, Mohan K, et al. Herpes simplex virus-2 (HSV-2) type-specific antibody correlates of protection in infants exposed to HSV-2 at birth. J Clin Invest 1992; 90(2):511-4.
11. Dudley KL, Bourne N, Milligan GN. Immune protection against HSV-2 in B-cell-deficient mice. Virology 2000; 270(2):454-63.
12. Mester JC, Milligan GN, Bernstein DI. The immunobiology of herpes simplex virus. In: Stanberry LR, editor. Genital and neonatal herpes. New York: John Wiley and Sons, Ltd.; 1996. p. 49-91.
13. Milligan GN, Bernstein DI. Analysis of herpes simplex virus-specific T cells in the murine female genital tract following genital infection with herpes simplex virus type 2. Virology 1995; 212(2):481-9.
14. Speck P, Simmons A. Precipitous clearance of herpes simplex virus antigens from the peripheral nervous systems of experimentally infected C57BL/10 mice. J Gen Virol 1998; 79(Pt 3):561-4.
15. Koelle DM, Posavad CM, Barnum GR, Johnson ML, Frank JM, Corey L. Clearance of HSV-2 from recurrent genital lesions correlates with infiltration of HSV-specific cytotoxic T lymphocytes. J Clin Invest 1998;101(7):1500-8.
16. Posavad CM, Koelle DM, Shaughnessy MF, Corey L. Severe genital herpes infections in HIV-infected individuals with impaired herpes simplex virus-specific CD8+ cytotoxic T lymphocyte responses. Proc Natl Acad Sci USA 1997; 94(19):10289-94.
17. Posavad CM, Koelle DM, Corey L. Tipping the scales of herpes simplex virus reactivation: the important responses are local. Nat Med 1998; 4(4):381-2.
18. Parr MB, Parr EL. Mucosal immunity in the female and male reproductive tracts. In: Ogra PL, LammME, Strober W, McGhee JR, Bienenstock J, editors. Handbook of mucosal immunology. San Diego: Academic Press; 1994. p. 677-89.
19. Parr MB, Parr EL. Mucosal immunity to herpes simplex virus type 2 infection in the mouse vagina is impaired by in vivo depletion of T lymphocytes. J Virol 1998; 72(4):2677-85.
20. Milligan GN, Bernstein DI, Bourne N. T lymphocytes are required for protection of the vaginal mucosae and sensory ganglia of immune mice against reinfection with herpes simplex virus type 2. J Immunol 1998; 160(12):6093-100.
21. Kuklin NA, Daheshia M, Chun S, Rouse BT. Role of mucosal immunity in herpes simplex virus infection. J Immunol 1998; 160(12):5998-6003.
22. Gallichan WS, Rosenthal KL. Long-term immunity and protection against herpes simplex virus type 2 in the murine female genital tract after mucosal but not systemic immunization. J Infect Dis 1998; 177(5):1155-61.
23. Neutra MR, Kozlowski PA. Mucosal vaccines: the promise and the challenge. Nat Rev Immunol 2006; 6(2):148-58.
24. Seder RA, Hill AV. Vaccines against intracellular infections requiring cellular immunity. Nature 2000; 406(6797):793-8.
25. Robinson HL, Montefiori DC, Johnson RP, Manson KH, Kalish ML, Lifson JD, et al. Neutralizing antibody-independent containment of immunodeficiency virus challenges by DNA priming and recombinant pox virus booster immunizations. Nat Med 1999; 5(5):526-34.
26. Yang K, Whalen BJ, Tirabassi RS, Selin LK, Levchenko TS, Torchilin VP, et al. A DNA vaccine prime followed by a liposome-encapsulated protein boost confers enhanced mucosal immune responses and protection. J Immunol 2008; 180(9):6159-67.
27. O'Hagan DT. Recent developments in vaccine delivery systems. Curr Drug Targets Infect Disord 2001; 1(3):273-86.
28. Alving CR, Koulchin V, Glenn GM, Rao M. Liposomes as carriers of peptide antigens: induction of antibodies and cytotoxic T lymphocytes to conjugated and unconjugated peptides. Immunol Rev 1995; 145:5-31.
29. Bourne N, Milligan GN, Schleiss MR, Bernstein DI, Stanberry LR. DNA immunization confers protective immunity on mice challenged intravaginally with herpes simplex virus type 2. Vaccine 1996; 14(13):1230-4.
30. Bernstein DI, Tepe ER, Mester JC, Arnold RL, Stanberry LR, Higgins T. Effects of DNA immunization formulated with bupivacaine in murine and guinea pig models of genital herpes simplex virus infection. Vaccine 1999; 17(15-16):1964-9.
31. Cattamanchi A, Posavad CM, Wald A, Baine Y, Moses J, Higgins TJ, et al. Phase I study of a herpes simplex virus type 2 (HSV-2) DNA vaccine administered to healthy, HSV-2 seronegative adults by a needle-free injection system. Clin Vaccine Immunol 2008;15(11):1638-43.
32. Posavad CM, Remington M, Mueller DE, Zhao L, Magaret AS, Wald A, et al. Detailed characterization of T cell responses to herpes simplex virus-2 in immune seronegative persons. J Immunol 2010;184(6):3250-9.
33. Pertmer TM, Roberts TR, Haynes JR. Influenza virus nucleoprotein-specific immunoglobulin G subclass and cytokine responses elicited by DNA vaccination are dependent on the route of vector DNA delivery. J Virol 1996; 70(9):6119-25.
34. Feltquate DM, Heaney S, Webster RG, Robinson HL. Different T helper cell types and antibody isotypes generated by saline and gene gun DNA immunization. J Immunol 1997; 158(5):2278-84.
35. Parr MB, Parr EL. The role of gamma interferon in immune resistance to vaginal infection by herpes simplex virus type 2 in mice. Virology 1999; 258(2): 282-94.
36. Milligan GN, Bernstein DI. Interferon-gamma enhances resolution of herpes simplex virus type 2 infection of the murine genital tract. Virology 1997; 229(1):259-68.
37. Baral RN, Saha A, Chatterjee SK, Foon KA, Krieg AM, Weiner GJ, et al. Immunostimulatory CpG oligonucleotides enhance the immune response of anti-idiotype vaccine that mimics carcinoembryonic antigen. Cancer Immunol Immunother 2003; 52(5):317-27.
38. Sandler AD, Chihara H, Kobayashi G, Zhu X, Miller MA, Scott DL, et al. CpG oligonucleotides enhance the tumor antigen-specific immune response of a granulocyte macrophage colony-stimulating factor-based vaccine strategy in neuroblastoma. Cancer Res 2003; 63(2):394-9.
39. Kutzler MA, Weiner DB. DNA vaccines: ready for prime time? Nat Rev Genet 2008; 9(10):776-88.
40. Lu S. Immunogenicity of DNA vaccines in humans: it takes two to tango. Hum Vaccin 2008; 4(6):449-52.
41. Domingo C, Gadea I, Pardeiro M, Castilla C, Fernandez S, Fernandez-Clua MA, et al. Immunological properties of a DNA plasmid encoding a chimeric protein of herpes simplex virus type 2 glycoprotein B and glycoprotein D. Vaccine 2003; 21(25-26):3565-74.
42. Bridges PA, Taylor KM. The effects of freeze-drying on the stability of liposomes to jet nebulization. J Pharm Pharmacol 2001; 53(3):393-8.
43. Bridges PA, Taylor KM. An investigation of some of the factors influencing the jet nebulisation of liposomes. Int J Pharm 2000;204(1---2):69-79.
44. Alving CR. Design and selection of vaccine adjuvants: animal models and human trials. Vaccine 2002; 20(Suppl. 3):S56-64.
45. Moynihan JS, Jones DH, Farrar GH, Howard CR. A novel microencapsulated peptide vaccine against hepatitis B. Vaccine 2001; 19(23-24):3292-300.
46. Natuk RJ, Cooper D, Guo M, Calderon P, Wright KJ, Nasar F, et al. Recombinant vesicular stomatitis virus vectors expressing herpes simplex virus type 2 gD elicit robust CD4+ Th1 immune responses and are protective in mouse and guinea pig models of vaginal challenge. J Virol 2006; 80(9):4447-57.

### SEQUENCES

Human codon-optimized HSV-2 gD nucleotide sequence (SEQ ID NO: 1) 
ATGGGACGGCTGACCAGCGGAGTGGGCACAGCCGCCCTGCTGGTCGTGGCTGTGGGCCTGCGCG
TGGTGTGCGCCAAGTACGCCCTGGCCGACCCCAGCCTGAAGATGGCCGACCCCAACCGGTTCCG
CGGCAAGAACCTGCCCGTGCTGGACCAGCTGACCGACCCTCCCGGCGTGAAGCGCGTGTACCAC
ATCCAGCCCAGCCTGGAGGACCCCTTCCAGCCCCCCAGCATCCCCATCACCGTGTACTACGCCG
TGCTGGAGCGCGCCTGCCGGAGCGTGCTGCTGCACGCCCCCAGCGAGGCCCCCCAGATTGTGCG
CGGAGCCAGCGACGAGGCCCGGAAGCACACCTACAACCTGACCATCGCCTGGTATCGGATGGGC
GACAACTGCGCCATCCCTATTACCGTGATGGAGTACACCGAGTGCCCCTACAACAAGAGCCTGG
GAGTGTGCCCCATCCGGACCCAGCCCCGGTGGAGCTACTACGACAGCTTCAGCGCTGTGAGCGA
GGACAACCTGGGCTTCCTGATGCACGCCCCTGCCTTCGAGACCGCCGGCACCTACCTGCGGCTG
GTGAAGATCAACGACTGGACCGAGATCACCCAGTTCATCCTGGAGCACCGCGCCAGAGCCAGCT
GCAAATACGCCCTGCCCCTGCGGATCCCCCCTGCCGCCTGCCTGACCAGCAAGGCCTATCAGCA
GGGCGTGACCGTGGACAGCATCGGCATGCTGCCCCGGTTCATCCCCGAGAACCAGCGGACCGTG
GCCCTGTACTCTCTGAAGATCGCCGGCTGGCACGGCCCCAAGCCCCCCTACACCAGCACCCTGC
TGCCCCCCGAGCTGAGCGACACCACCAACGCCACCCAGCCCGAGCTGGTGCCCGAGGACCCCGA
GGATAGCGCCCTGCTGGAGGATCCCGCCGGAACAGTGAGCAGCCAGATCCCCCCCAACTGGCAC
ATCCCTAGCATCCAGGACGTGGCCCCCCACCACGCCCCAGCCGCCCCTAGCAACCCCGGCCTGA
TCATCGGCGCCCTGGCCGGCAGCACCCTGGCCGCCCTGGTGATCGGCGGCATCGCCTTTTGGGT
GCGCAGACGCGCCCAGATGGCCCCCAAGCGGCTGCGGCTGCCCCACATCCGCGACGACGACGCC
CCTCCATCTCACCAGCCCCTGTTCTAG
Truncated gD amino acid sequence-amino acids 1-314 (SEQ ID NO: 2) 
M G R L T S G V G T A A L L V V A V G L R V V C A K Y A L A D P
S L K M A D P N R F R G K N L P V L D Q L T D P P G V K R V Y H
I Q P S L E D P F Q P P S I P I T V Y Y A V L E R A C R S V L L
H A P S E A P Q I V R G A S D E A R K H T Y N L T I A W Y R M G
D N C A I P I T V M E Y T E C P Y N K S L G V C P I R T Q P R W
S Y Y D S F S A V S E D N L G F L M H A P A F E T A G T Y L R L
V K I N D W T E I T Q F I L E H R A R A S C K Y A L P L R I P P
A A C L T S K A Y Q Q G V T V D S I G M L P R F I P E N Q R T V
A L Y S L K I A G W H G P K P P Y T S T L L P P E L S D T [?] N A
T Q P E L V P E D P E D S A L L E D P A G T V S S Q
Full-length gD amino acid sequence (SEQ ID NO: 3) 
M G R L T S G V G T A A L L V V A V G L R V V C A K Y A L A D P
S L K M A D P N R F R G K N L P V L D Q L T D P P G V K R V Y H
I Q P S L E D P F Q P P S I P I T V Y Y A V L E R A C R S V L L
H A P S E A P Q I V R G A S D E A R K H T Y N L T I A W Y R M G
D N C A I P I T V M E Y T E C P Y N K S L G V C P I R T Q P R W
S Y Y D S F S A V S E D N L G F L M H A P A F E T A G T Y L R L
V K I N D W T E I T Q F I L E H R A R A S C K Y A L P L R I P P
A A C L T S K A Y Q Q G V T V D S I G M L P R F I P E N Q R T V
A L Y S L K I A G W H G P K P P Y T S T L L P P E L S D T T N A
T Q P E L V P E D P E D S A L L E D P A G T V S S Q I P P N W H
I P S I Q D V A P H H A P A A P S N P G L I I G A L A G S T L A
A L V I G G I A F W V R R R A Q M A P K R L R L P H I R D D D A
P P S H Q P L F
Full-length gD amino acid sequence variant #1 (SEQ ID NO: 4) 
MGRLTSGVGTAALLVVAVGLRVVCAKYALADPSLKMADPNRFRGKNLPVLDQLTDPPGVKRVYH
IQPSLEDPFQPPSIPITVYYAVLERACRSVLLHAPSEAPQIVRGASDEARKHTYNLTIAWYRMG
DNCAIPITVMEYTECPYNKSLGVCPIRTQPRWSYYDSFSAVSEDNLGFLMHAPAFETAGTYLRL
VKINDWTEITQFILEHRARASCKYALPLRIPPAACLTSKAYQQGVTVDSIGMLPRFIPENQRTV
ALYSLKIAGWHGPKPPYTSTLLPPELSDTTNATQPELVPEDPEDSALLEDPAGTVSSQIPPNWH
IPSIQDVAPHHAPAAPSNPGLIIGALAGSTLAVLVIGGIAFWVRRRAQMAPKRLRLPHIRDDDA
PPSHQPLFY
Full-length gD amino acid sequence variant #2 (SEQ ID NO: 5) 
MGRLTSGVGTAALLVVAVGLRVVCAKYALADPSLKMADPNRFRGKNLPVLDRLTDPPGVKRVYH
IQPSLEDPFQPPSIPITVYYAVLERACRSVLLHAPSEAPQIVRGASDEARKHTYNLTIAWYRMG
DNCAIPITVMEYTECPYNKSLGVCPIRTQPRWSYYDSFSAVSEDNLGFLMHAPAFETAGTYLRL
VKINDWTEITQFILEHRARASCKYALPLRIPPAACLTSKAYQQGVTVDSIGMLPRFIPENQRTV
ALYSLKIAGWHGPKPPYTSTLLPPELSDTTNATQPELVPEDPEDSALLEDPAGTVSSQIPPNWH
IPSIQDVAPHHAPAAPSNPGLIIGALAGSTLAVLVIGGIAFWVRRRAQMAPKRLRLPHIRDDDA
PPSHQPLFY
Full-length gD amino acid sequence variant #3 (SEQ ID NO: 6) 
MGRLTSGVGTAALLVVAVGLRVVCAKYALADPSLKMADPNRFRGKNLPVLDQLTDPPGVKRVYH
IQPSLEDPFQPPSIPITVYYAVLERACRSVLLHAPSEAPQIVRGASDEARKHTYNLTIAWYRMG
DNCAIPITVMEYTECPYNKSLGVCPIRTQPRWSYYDSFSAVSEDNLGFLIHAPAFETAGTYLRL
VKINDWTEITQFILEHRARASCKYALPLRIPPAACLTSKAYQQGVTVDS[?]GMLPRFIPENQRTV
 
ALYSLKIAGWHGPKPPYTSTLLPPELSDTTNATQPELVPEDPEDSALLEDPAGTVSSQIPPNWH
IPSIQDVAPHHAPAAPSNPGLIIGALAGSTLAVLVIGGIAFWVRRRAQMAPKRLRLPHIRDDDA
PPSHQPLFY
Full-length gD amino acid sequence variant #4 (SEQ ID NO: 7)

MGRLTSGVGTAALLVVAVGLRVVCAKYALADPSLKMADPNRFRGKNLPVLDQLTDPPGVKRVYH
IQPSLEDPFQPPSIPITVYYAVLERACRSVLLHAPSEAPQIVRGASDEARKHTYNLTIAWYRMG
DNCAIPITVMEYTECPYNKSLGVCPIRTQPRWSYYDSFSAVSEDNLGFLMHAPAFETAGTYLRL
VKINDWTEITQFILEHRARASCKYALPLRIPPAACLTSKAYQQGVTVDSIGMLPRFIPENQRTV
ALYSLKIAGWHGPKPPYTSTLLPPELSDTTNATQPELVPEDPEDSALLEDPAGTVSSQ[?]PPNWH
IPSIQDVAPHHAPAAPSNPGLIIGALAGSTLAVLVIGGIAFWVRRRAQMAPKRLRLPHIRDDDA
PPSHQPLFY

## Claims

1. A priming preparation and a boosting preparation for use in a method for eliciting a protective immune response against HSV-2 in a mammal, the method comprising administering the priming preparation and the boosting preparation to the mammal:
(a) wherein the priming preparation comprises a vector encoding an HSV-2 antigen under the control of a promoter, wherein the antigen is a full-length HSV-2 gD glycoprotein, and wherein the sequence encoding the gD glycoprotein is codon-optimized for expression in mammalian cells; and
(b) wherein the boosting preparation comprises an extracellular domain of the gD glycoprotein encapsulated in liposomes,
wherein in the method the priming preparation is administered intramuscularly and the boosting preparation is administered mucosally, thereby eliciting the protective immune response in the mammal.

2. The priming preparation and the boosting preparation for use according to claim 1,
wherein the gD glycoprotein sequence is codon-optimized for expression in human cells.

3. The priming preparation and the boosting preparation for use according to claim 1,
wherein the vector encodes a full-length gD glycoprotein sequence comprising the sequence of SEQ ID NO: 1.

4. The priming preparation and the boosting preparation for use according to claim 1,
wherein the extracellular domain of the gD glycoprotein comprises a sequence of SEQ ID NO: 2.

5. The priming preparation and the boosting preparation for use according to claim 1,
wherein the liposomes are anionic liposomes.

6. The priming preparation and the boosting preparation for use according to claim 1,
wherein the boosting preparation is administered intranasally.

7. The priming preparation and the boosting preparation for use according to claim 1,
wherein one or more symptoms of HSV-2 infection are ameliorated in the mammal following administration of the boosting preparation.

8. The priming preparation and the boosting preparation for use according to claim 7,
wherein one or more symptoms of HSV-2 infection is viral shedding.

9. The priming preparation and the boosting preparation for use according to claim 6,
wherein viral vaginal load in the mammal is reduced.

## Patentansprüche

1. Priming-Präparat und Boosting-Präparat zur Verwendung in einem Verfahren zum Hervorrufen einer schützenden Immunreaktion gegen HSV-2 in einem Säugetier, wobei das Verfahren das Verabreichen des Priming-Präparats und des Boosting-Präparats an das Säugetier umfasst:
(a) wobei das Priming-Präparat einen für ein HSV-2-Antigen kodierenden Vektor unter der Kontrolle eines Promotors umfasst, wobei das Antigen ist ein HSV-2-gD-Glykoprotein voller Länge ist und wobei die für das gD-Glykoprotein kodierende Sequenz für die Expression in Säugetierzellen Codon-optimiert ist; und
(b) wobei das Boosting-Präparat eine extrazelluläre Domäne des gD-Glykoproteins, verkapselt in Liposomen umfasst,
wobei in dem Verfahren das Priming-Präparat intramuskulär und das Boosting-Präparat mukosal verabreicht wird, wodurch die schützende Immunreaktion in dem Säugetier hervorgerufen wird.

2. Priming-Präparat und Boosting-Präparat zur Verwendung nach Anspruch 1, wobei die gD-Glykoproteinsequenz für die Expression in menschlichen Zellen Codon-optimiert ist.

3. Priming-Präparat und Boosting-Präparat zur Verwendung nach Anspruch 1, wobei der Vektor für eine gD-Glykoproteinsequenz voller Länge kodiert, die die Sequenz von Seq.-ID Nr. 1 umfasst.

4. Priming-Präparat und Boosting-Präparat zur Verwendung nach Anspruch 1, wobei die extrazelluläre Domäne des gD-Glykoproteins eine Sequenz von Seq.-ID Nr. 2 umfasst.

5. Priming-Präparat und Boosting-Präparat zur Verwendung nach Anspruch 1, wobei die Liposomen anionische Liposomen sind.

6. Priming-Präparat und Boosting-Präparat zur Verwendung nach Anspruch 1, wobei das Boosting-Präparat intranasal verabreicht wird.

7. Priming-Präparat und Boosting-Präparat zur Verwendung nach Anspruch 1, wobei ein oder mehrere Symptome einer HSV-2-Infektion bei dem Säugetier infolge der Verabreichung des Boosting-Präparats gelindert werden.

8. Priming-Präparat und Boosting-Präparat zur Verwendung nach Anspruch 7, wobei ein oder mehrere Symptome der HSV-2-Infekion Virusfreisetzung sind.

9. Priming-Präparat und Boosting-Präparat zur Verwendung nach Anspruch 6, wobei die vaginale Virusbelastung in dem Säugetier reduziert wird.

## Revendications

1. Préparation d'amorçage et préparation de stimulation destinées à être utilisées dans un procédé pour provoquer une réponse protectrice immunitaire contre le Virus de l'Herpès Simplex de type 2 (HSV-2) chez un mammifère, le procédé comprenant l'administration de la préparation d'amorçage et de la préparation de stimulation au mammifère .
(a) dans lesquelles la préparation d'amorçage comprend un vecteur codant pour un antigène HSV-2 sous la commande d'un promoteur, l'antigène étant une glycoprotéine HSV-2 gD de longueur totale, et dans lesquelles la séquence codant pour la glycoprotéine gD est optimisée en termes de codons pour une expression dans des cellules mammifères ; et
(b) dans lesquelles la préparation de stimulation comprend un domaine extracellulaire de la glycoprotéine gD encapsulé dans des liposomes,
dans lesquelles, dans le procédé, la préparation d'amorçage est administrée par voie intramusculaire et la préparation de stimulation est administrée par voie muqueuse, afin de provoquer la réponse protectrice immunitaire chez le mammifère.

2. Préparation d'amorçage et préparation de stimulation pour une utilisation selon la revendication 1, dans lesquelles la séquence de glycoprotéine gD est optimisée en termes de codons pour une expression dans des cellules humaines.

3. Préparation d'amorçage et préparation de stimulation pour une utilisation selon la revendication 1, dans lesquelles le vecteur code pour une séquence de glycoprotéine gD de longueur totale comprenant la séquence de la SEQ ID NO : 1.

4. Préparation d'amorçage et préparation de stimulation pour une utilisation selon la revendication 1, dans lesquelles le domaine extracellulaire de la glycoprotéine gD comprend une séquence de la SEQ ID NO : 2.

5. Préparation d'amorçage et préparation de stimulation pour une utilisation selon la revendication 1, dans lesquelles les liposomes sont des liposomes anioniques.

6. Préparation d'amorçage et préparation de stimulation pour une utilisation selon la revendication 1, dans lesquelles la préparation de stimulation est administrée par vois intranasale.

7. Préparation d'amorçage et préparation de stimulation pour une utilisation selon la revendication 1, dans lesquelles un ou plusieurs symptômes d'infection HSV-2 sont améliorés chez les mammifères après administration de la préparation de stimulation.

8. Préparation d'amorçage et préparation de stimulation pour une utilisation selon la revendication 7, dans lesquelles un ou plusieurs symptômes d'infection HSV-2 est une excrétion virale.

9. Préparation d'amorçage et préparation de stimulation pour une utilisation selon la revendication 6, dans lesquelles une charge virale vaginale chez le mammifère est réduite.
